# EUROPEAN PATENT APPLICATION

(11) **EP 4 071 241 A1**
(43) Date of publication of application: **12.10.2022**
(21) Application number: 19955117.7
(22) Date of filing: 06.12.2019
(51) Int. Cl.: C12N 9/02, C12P 7/64, A23L 33/115, A61K 8/365, A61K 31/202, A61P 17/00

(54) **MONO-HYDROXY OR DI-HYDROXY DERIVATIVES OF POLYUNSATURATED FATTY ACIDS, PRODUCTION METHOD THEREFOR, AND USE THEREOF**

(71) Applicant: Korea Research Institute of Bioscience and Biotechnology, Daejeon 34141 (KR); Kolon Industries, Inc., Seoul 07793 (KR)
(72) Inventor: SEO, Jeong Woo, Daejeon 34141 (KR); YI, Jong Jae, Daejeon 34141 (KR); HEO, Sun Yeon, Daejeon 34141 (KR); JU, Jung Hyun, Daejeon 34141 (KR); KIM, Chul Ho, Daejeon 34141 (KR); OH, Baek Rock, Daejeon 34141 (KR); LEE, Gil Yong, Hwaseong-si Gyeonggi-do 18321 (KR); LEE, Kyung Min, Seoul 04343 (KR); CHO, Hee Won, Incheon 22760 (KR)
(74) Representative: Ter Meer Steinmeister & Partner
(86) International application number: PCT/KR2019/017246
(87) International publication number: WO 2021/112310

(57) **Abstract**

The present invention relates to an enzyme for producing hydroxy derivatives of polyunsaturated fatty acids, a method for producing hydroxy derivatives of polyunsaturated fatty acids using the same, and an SPMs complex composition containing the hydroxy derivatives of polyunsaturated fatty acids.

The enzyme may produce hydroxy derivatives of polyunsaturated fatty acids in a single reaction, and thus may be very usefully used for *in vitro* production. In addition, the SPMs complex composition containing the hydroxy derivatives of polyunsaturated fatty acids promotes collagen production, inhibits the production of NO and expression of TNF-α and IL-6, increases the expression of filaggrin and loricrin, and thus may be usefully used for preventing or alleviating skin aging or wrinkles, strengthening skin barrier, or preventing, alleviating or treating skin diseases comprising atopic dermatitis.

<110>    Korea Research Institute of Bioscience & Biotechnology
         KOLON LIFE SCIENCE INC.

<120>    MONO-HYDROXY OR DI-HYDROXY DERIVATIVES OF POLYUNSATURATED FATTY
         ACID, THE PRODUCTION THEREOF, AND USES THEREOF

<130>    2019OPA3336

<160>    8

<170>    KoPatentIn 3.0

<210>    1
<211>    727
<212>    PRT
<213>    Artificial Sequence

<220>
<223>    enzyme (K1)


<400>    1
Met Ser Val Ser Leu Met Gln Ala Tyr Thr Val Met Asn Ser Ala Thr
  1               5                  10                  15

Val Lys Lys Thr Leu Leu Asn Leu Tyr Lys Leu Val Val Thr Ile Arg
             20                  25                  30

Lys Gly Leu Gly Ser Glu Ala Tyr Gln Tyr Asn Tyr Asn Tyr Ile Pro
         35                  40                  45

Pro Leu Ala Met Thr Glu Gly Pro Pro Leu Leu Asp Ser Gly Leu His
     50                  55                  60

Leu Pro Leu Glu Glu Leu Pro Asn Pro Lys Trp Ile Phe Leu Val Leu
 65                  70                  75                  80

His Lys Leu Ala Val Ile Thr Val Asn Lys Ala Ile Gly Lys Leu Val
                 85                  90                  95

Thr Glu Gly Ser Glu Leu Val Ser Phe Ser Val Thr Glu Gly Pro Glu
            100                 105                 110

Ala Asp Ile Asp Leu Leu Asn Glu Lys Ala Gln Ala Leu Glu Glu Leu
        115                 120                 125

Glu Gln Gln Ile Gln Ser Ala Asp Ser Glu Gln Asp Leu Glu Gln Val
    130                 135                 140

Ala Leu Gln Leu Ala Ala Leu Thr Arg Glu Ile Ser Asp Glu Leu Asp
145                 150                 155                 160

Pro Val Asp Ser Ser Ser Val Ser Phe Asp Ile Ser Ser Ala Pro Glu
                165                 170                 175

Ser Glu Leu Glu Ala Gln Ile Arg Glu Ile Asp Glu Gln Val Ala Ser
            180                 185                 190

Ser Glu Glu Val Ser Phe Ser Val Pro Ser Gly Thr Glu Glu Ser Glu
        195                 200                 205

Gly Ile Ser Asp Ser Leu Leu Ala Ala Phe Arg Glu Ala Ile Thr Ser
    210                 215                 220

Ser Gly Lys Leu Ile Glu Leu Asp Glu Gln Gln Ala Thr Ser Gly Glu
225                 230                 235                 240

Val Ser Phe Ser Val Asp Ser Phe Ala Ala Ala Pro Ser Leu Glu Asp
                245                 250                 255

Tyr Asn Lys Leu Phe Thr Arg Ile Ser Leu Pro Lys Ile Ser Ala Arg
            260                 265                 270

Phe Gln Glu Asp Gln Val Phe Ala Tyr Met Gln Val Ala Gly Pro Asn
        275                 280                 285

Ala Val Met Leu Glu Gln Val Ala Glu Gly Asp Pro Phe Val Val Arg
    290                 295                 300

Leu Thr Gln Glu Leu Pro Glu Gly Gly Tyr Ala Lys Ile Ile Gly Thr
305                 310                 315                 320

Gly Asp Ser Leu Ala Ala Ala Val Lys Glu Gly Arg Val Tyr Lys Ala
                325                 330                 335

Asp Tyr Ser Leu Leu Glu Gly Met Glu Asn Gly Thr Phe Pro Asp Gln
            340                 345                 350

Gln Lys Tyr Thr Tyr Ala Pro Leu Gly Leu Phe Ala Val Pro Pro Gln
        355                 360                 365

Gly Cys Ser Ser Arg Asn Leu Leu Pro Val Ala Ile Gln Cys Gln Pro
    370                 375                 380

Gly Ala Asp Cys Thr Gly Pro Val Phe Thr Pro Leu Lys Gly Glu Ser
385                 390                 395                 400

Trp Met Val Ala Lys Thr Ile Leu Gln Met Ala Asn Val Asn Tyr Leu
                405                 410                 415

Glu Leu Val Ser His Leu Gly Gln Thr His Leu Val Ile Glu Pro Phe
            420                 425                 430

Val Leu Ala Thr Lys Arg Ile Leu Pro Arg Thr His Arg Leu Arg Leu
        435                 440                 445

Leu Leu Asp Pro His Phe Glu Gly Thr Val Leu Ile Asn Tyr Gly Ala
    450                 455                 460

His Lys Thr Leu Ile Ala Pro Gly Trp Asp Val Asp Lys Leu Leu Ala
465                 470                 475                 480

Ser Thr Ile Glu Asn Asp Gly Leu Ile Ala Val Lys Gly Ala Gln Ser
                485                 490                 495

His Leu His Asn Phe Asn Glu Val Ser Phe Pro Lys Leu Leu Ala Ser
            500                 505                 510

Arg Gly Val Thr Asn Ser Ala Gln Leu Pro Glu Tyr Pro Tyr Arg Asp
        515                 520                 525

Asp Gly Leu Leu Ile Trp Asn Ala Leu His Glu Trp Val Ser Asp Tyr
    530                 535                 540

Leu Arg Leu Cys Tyr Thr Ser Asp Ala Gln Ile Val Ala Asp Ala Asp
545                 550                 555                 560

Leu Gln Ser Trp Ala Lys Glu Leu Val Ser Asp Ala Gly Gly Arg Leu
                565                 570                 575

Lys Asn Phe Gly Glu Asp Ser Ser Gly Ala Ile Lys Thr Leu Asp Tyr
            580                 585                 590

Leu Ile Asp Thr Val Cys Ser Val Ile Phe Ile Ala Ser Ala Gln His
        595                 600                 605

Ala Ala Met Asn Phe Pro Gln Asn Glu Leu Met Thr Tyr Ala Pro Ala
    610                 615                 620

Phe Pro Leu Ala Val Tyr Ser Pro Ala Pro Thr Asn Pro Asp Glu Ser
625                 630                 635                 640

Met Asp Trp Met Glu Trp Leu Pro Ser Leu Glu Lys Ala Glu Asn Gln
                645                 650                 655

Val Lys Val Cys Tyr Leu Leu Gly Ala Val Tyr Tyr Thr Gln Leu Gly
            660                 665                 670

Gly Tyr Gly Lys Lys Gln Phe Thr Asp Ser Lys Val Lys Ala Ala Leu
        675                 680                 685

Gly Lys Phe Gln Asn Arg Leu Lys Asp Ile Glu Lys Glu Ile Thr Lys
    690                 695                 700

Lys Asn Ser Ser Arg Ile Met Pro Tyr Lys Phe Leu Leu Pro Ser Gln
705                 710                 715                 720

Ile Pro Gln Ser Ile Asn Ile
                725


<210>    2
<211>    571
<212>    PRT
<213>    Artificial Sequence

<220>
<223>    enzyme (K2)


<400>    2
Met Val Asp Asn Met Lys Pro Ser Leu Pro Gln Asp Asp Pro Asn Gln
1               5                   10                  15

Glu Gln Arg Lys Asp Ser Leu Asn Arg Gln Gln Gln Ala Tyr Gln Phe
            20                  25                  30

Asp Tyr Glu Ser Leu Ser Pro Leu Ala Leu Leu Lys Asn Val Pro Ala
        35                  40                  45

Val Glu Asn Phe Ser Ser Lys Tyr Ile Gly Glu Arg Ile Leu Ala Thr
    50                  55                  60

Ser Glu Leu Pro Ala Asn Met Leu Ala Ala Asp Ser Arg Thr Phe Leu
65                  70                  75                  80

Asp Pro Leu Asp Glu Leu Gln Asp Tyr Glu Asp Phe Phe Thr Leu Leu
                85                  90                  95

Pro Leu Pro Ala Val Ala Lys Ile Tyr Gln Thr Asp Arg Ser Phe Ala
            100                 105                 110

Glu Gln Arg Leu Ser Gly Ala Asn Pro Met Val Leu Arg Leu Leu Asp
        115                 120                 125

Ala Gly Asp Pro Arg Ala Gln Thr Leu Ala Gln Ile Ser Ser Phe His
    130                 135                 140

Pro Leu Phe Asp Leu Gly Gln Glu Leu Gln Gln Lys Asn Ile Tyr Val
145                 150                 155                 160

Ala Asp Tyr Thr Gly Thr Asp Glu His Tyr Arg Ala Pro Ser Lys Ile
                165                 170                 175

Gly Gly Gly Ser Tyr Glu Lys Gly Arg Lys Phe Leu Pro Lys Pro Arg
            180                 185                 190

Ala Phe Phe Ala Trp Arg Trp Thr Gly Ile Arg Asp Arg Gly Glu Met
        195                 200                 205

Thr Pro Ile Ala Ile Gln Leu Asp Pro Thr Pro Asp Ser His Val Tyr
    210                 215                 220

Thr Pro Phe Asp Pro Pro Val Asp Trp Leu Phe Ala Lys Leu Cys Val
225                 230                 235                 240

Gln Val Ala Asp Ala Asn His His Glu Met Ser Ser His Leu Gly Arg
                245                 250                 255

Thr His Leu Val Met Glu Pro Ile Ala Ile Val Thr Ala Arg Gln Leu
            260                 265                 270

Ala Gln Asn His Pro Leu Ser Leu Leu Leu Lys Pro His Phe Arg Phe
        275                 280                 285

Met Leu Thr Asn Asn Glu Leu Ala Arg Ser Tyr Leu Ile Ala Pro Gly
    290                 295                 300

Gly Pro Val Asp Glu Leu Leu Gly Gly Thr Leu Pro Glu Thr Met Glu
305                 310                 315                 320

Ile Ala Arg Glu Ala Cys Ser Thr Trp Ser Leu Asp Glu Phe Ala Leu
                325                 330                 335

Pro Ala Glu Leu Lys Asn Arg Gly Met Asp Asp Thr Asn Gln Leu Pro
            340                 345                 350

His Tyr Pro Tyr Arg Asp Asp Gly Leu Leu Leu Trp Asp Ala Ile Glu
        355                 360                 365

Thr Phe Val Ser Gly Tyr Leu Lys Phe Phe Tyr Pro Thr Glu Ile Ala
    370                 375                 380

Ile Val Gln Asp Val Glu Leu Gln Thr Trp Ala Gln Glu Leu Ala Ser
385                 390                 395                 400

Asp Arg Gly Gly Lys Val Lys Gly Met Pro Pro Arg Ile Asn Thr Val
                405                 410                 415

Glu Gln Leu Ile Lys Ile Val Thr Thr Ile Ile Phe Thr Cys Gly Pro
            420                 425                 430

Gln His Ser Ala Val Asn Phe Pro Gln Tyr Glu Tyr Met Ser Phe Ala
        435                 440                 445

Ala Asn Met Pro Leu Ala Ala Tyr Arg Asp Ile Pro Lys Ile Thr Ala
    450                 455                 460

Ser Gly Asn Leu Glu Val Ile Thr Glu Lys Asp Ile Leu Arg Leu Leu
465                 470                 475                 480

Pro Pro Tyr Lys Arg Ala Ala Asp Gln Leu Lys Ile Leu Phe Thr Leu
                485                 490                 495

Ser Ala Tyr Arg Tyr Asp Arg Leu Gly Tyr Tyr Asp Lys Ser Phe Arg
            500                 505                 510

Glu Leu Tyr Arg Met Ser Phe Asp Glu Val Phe Ala Gly Thr Pro Ile
        515                 520                 525

Gln Leu Leu Ala Arg Gln Phe Gln Gln Asn Leu Asn Met Ala Glu Gln
    530                 535                 540

Lys Ile Asp Ala Asn Asn Gln Lys Arg Val Ile Pro Tyr Ile Ala Leu
545                 550                 555                 560

Lys Pro Ser Leu Val Ile Asn Ser Ile Ser Met
                565                 570

<210>    3
<211>    2580
<212>    DNA
<213>    Artificial Sequence

<220>
<223>    enzyme (K1)

<400>    3
atgacaggtg ggatgtttgg acgtaagggg cagaagatta aggggacagt ggtattgatg      60

cctaagaatg tgttggattt caacgccata acctccgtcg gcaagggcag tgctaaggac     120

accgccaccg atttcttggg caagggcttg gacgcactgg gtcacgcagt tgatgccctc     180

actgccttcg ctggccacag catctccttg cagcttatct cggctactca gactgatggt     240

agtggaaaag gtaaagttgg aaacgaagcc tatttggaaa agcatcttcc gaccttgcct     300

acgctgggag cgcgccagga agcgttcgat attaactttg aatgggatgc gagttttgga     360

attccaggag cattttacat caaaaatttt atgacggatg agtttttcct cgtctctgtt     420

aaattagagg acattccgaa ccatggaacc attaacttcg tttgtaactc atgggtttat     480

aacttcaaat cttacaagaa gaatcgcatt ttctttgtca atgataccta tctaccgtcc     540

gcgacgccag gcccactagt taagtacaga caggaagaat tggaggtttt acggggcgat     600

ggcacgggga agcgtcgcga ctttgaccgg atctatgatt atgatatcta caatgatttg     660

ggcaatccag atggtggtga tccgcgccca atcattgggg gcagcagcaa ctatccttac     720

cctcgccgtg ttcggaccgg tagagaaaag accaggaaag accccaacag tgagaaacca     780

ggcgagatat atgttccgcg cgatgaaaac ttcggtcact taaagtcatc tgatttcctt 840

acgtacggga tcaagtcctt atctcagaac gtgattcctt tgttcaaatc tataatactg 900

aacttacgag tcacatcgtc ggagttcgat tcgttcgacg aagtgcgtgg tctctttgaa 960

ggtggaatca agctgccaac aaatatactg agccaaatta gcccattacc ggtcctcaag 1020

gaaatcttcc gcactgatgg tgaaaatacc cttcaatttc caccgccgca tgtaatccga 1080

gttagtaaat ctggatggat gactgacgac gagtttgcca gagaaatgat tgctggtgta 1140

aatcccaatg taattcgtcg tttgcaagag ttcccaccaa aaagcactct ggatcccgca 1200

acctatggtg atcagactag taccataacg aaacagcagt tggagattaa cttgggtggg 1260

gtcacagtag aagaggcgat ttcggctcac agattattta tcttagacta ccatgacgca 1320

ttctttccgt atctaacgaa gatcaacagc ttgcctattg caaaagctta tgccacccgg 1380

acaatcctgt tcctgaaaga cgacggatct ctaaagccgc ttgctatcga attaagcaaa 1440

cctgcgacag tgagtaaagt ggtgttgcct gcgacagagg gtgttgagag tacaatatgg 1500

ttgctcgcca aggcgcatgt cattgtgaat gactctggtt atcatcaatt aataagccat 1560

tggttaaata cccatgcagt catggaacca tttgccattg ccacgaaccg ccatctcagt 1620

gtgcttcacc ccatttataa acttctttac cctcactaca aggacactat aaatatcaat 1680

ggccttgcta ggcagtccct gattaacgcg ggtggcatta ttgagcagac ctttttgcct 1740

ggaaaatact ccattgaaat gtcatcagtt gtttacaaga attgggtttt cactgaccag 1800

gcactgcccg cggatcttgt caagcgcgga ttggcagttg aggatccctc tgccccgcat 1860

ggtcttcgcc tggtgattga agactatccg tatgcagttg atggacttga aatctgggat 1920

gcaattaaaa cctgggtcca tgagtatgtc tctgtgtatt acccgacaaa tgcggccatt 1980

caacaggaca ctgaacttca agcatggtgg aaagaagtag tggagaaggg tcatggtgac 2040

ttaaaagata agccttggtg gcctaaatta cagactgtgg aagatctcat tcaatcctgc 2100

tctataatta tatggacagc ttcggctctc cacgcagctg tcaattttgg ccaatacccc 2160

tatggcggtt atatcgtgaa ccgtccaacc cttgcacgta ggtttatccc agaagaagga 2220

accaaagaat atgatgagat ggtgaaagat cctcagaagg catatctgag aacaatcaca 2280

cccaagttcg agacccttat tgacatttca gtgatagaga tattatcacg gcacgcttct 2340

gatgaagtct atctgggcca acgtgataat ccaaattgga ctacggattc aaaggcactg 2400

gaagctttca aaaagtttgg aaacaaactg gccgaaattg agggaaaaat cacacagcgg 2460

aacaatgatc caagtctgaa aagccgacat gggccagttc agcttccata cacgttgctg 2520

catcgttcaa gcgaagaagg gatgagtttc aaaggaattc ccaacagtat ttccatctaa 2580

2580

<210>     4
<211>     2595
<212>     DNA
<213>     Artificial Sequence

<220>
<223>     enzyme (K2)


<400>     4
atgtttggca tcttcgacaa ggggcagaag ataaagggga ccgtggtgtt gatgccgaag          60

aacgtgttgg acttcaacgc cataacctcc atcggtaaag gtggtgttat tgacacagcc         120

accggcatct taggccaggg cgttagctta gttggtggag taattgatac cgccacttcc         180

tttttaggcc gtaatatctc catgcaattg atcagtgcta ctcagaccga tggctctgga         240

aatgggaaag taggaaaaga agtctatttg gaaaaacatc ttccgaccct accaacgttg         300

ggagcacgtc aggatgcgtt tagtattttc tttgagtggg acgcttcgtt tggaattccg         360

ggagcatttt atatcaaaaa ctttatgacg gatgagttct tcctcgtcag tgttaaactc         420

gaagacattc cgaaccatgg aaccattgaa tttgtttgca actcatgggt ttacaacttc         480

cgatcttaca aaaagaatcg gattttcttt gtcaatgaca catatcttcc aagtgccaca         540

ccagctccgc tgcttaagta ccgcaaagaa gaattggagg ttttaagagg agatggtaca         600

ggaaagcgca aggactttga cagaatctat gattatgatg tctataatga tttgggcaat         660

ccagatggtg gtgatcctcg cccaattctt ggtgggtcta gcatctatcc ctaccctcgc         720

cgggttcgta cagggagaga aaggacccgt accgatccca actcagagaa accgggcgag         780

gtttatgttc cacgcgacga aaattttggt cacctgaagt catcggattt ccttacatac         840

ggaatcaaat ctctgtccca cgatgtgata cctttgttca aaagcgcgat ttttcagtta          900

cgggtcacat cgagtgagtt tgaaagcttc gaagatgtgc gttcacttta cgagggtgga         960

attaaattgc caacagatat actgagccaa attagcccct taccagccct caaagaaatc        1020

tttcgcactg acggtgaaaa tgttcttcag tttccacctc cacatgtagc caaagtttcc        1080

aagtctgggt ggatgactga tgaggaattt gctcgtgagg tgattgccgg cgtaaaccca        1140

aatgtcatcc gtcggcttca ggaattcccg ccaaagagca cacttgatcc cacactctat        1200

ggtgatcaga ccagcaccat aacaaaagaa cagttggaaa ttaacatggg tggggtcaca        1260

gtagaagagg cgcttagtac ccagcggtta ttcatactgg actatcaaga tgcatttatc        1320

ccatatttga cgcggataaa cagcctacct actgcaaaag cgtatgccac aaggacaatc        1380

ctgttcttga aagacgatgg cactttgaag ccattagcta tcgaattaag caaaccacat        1440

ccagatggag ataatttggg tcctgagtcg atagtggtgc tgcctgcaac ggaaggtgtt        1500

gatagtacca tctggctatt ggccaaggct catgttattg tgaatgactc tggttatcac        1560

cagctggtaa gccattggtt aaacactcat gcagtgatgg aaccatttgc cattgcaacc 1620

aataggcacc tcagtgtgct ccaccctatt tataaacttt tgtatcctca ctaccgcgac 1680

accataaata ttaatggctt ggctcgccag tccctgatta atgcggatgg cattatcgaa 1740

aaatcatttt tgcccggaaa gtactccatt gagatgtctt catcagtcta caaaaattgg 1800

gtttttactg accaggcatt accagccgat ctggtcaagc gaggattggc aattgaagat 1860

ccctctgcac cccatggcct tcgccttgtg atagaggact acccttatgc tgttgatgga 1920

cttgaaatat gggatgctat taagacatgg gttcatgaat atgtctcctt gtattaccca 1980

acagatgcag ccgttcaaca ggacactgaa ctacaagcat ggtggaagga agcggtggag 2040

aagggtcacg gtgacctgaa agaaaagcct tggtggccta aaatgcagac aacggaagat 2100

ctgattcaat catgttctat tatcgtttgg acagcttcgg ctctccatgc tgcggttaat 2160

tttggacaat atccttacgg gggcttaatc ctgaaccgtc caactctagc ccgccgtttt 2220

atcccagcgg agggaacccc agagtatgat gaaatggtga agaatcctca aaaagcttat 2280

ctgcgaacaa tcacacccaa gtttgagacc ctgattgatt tatcggtaat tgaaatattg 2340

tcacgacatg cttctgatga gatatacctg ggagagcgcg aaaccccaaa ttggacgact 2400

gataagaagg cattagaagc attcaaacgt tttggcagca aactgactgg tattgaagga 2460

aaaatcaatg cgaggaacag tgatccgagt cttagaaacc ggacggggcc agttcagctg 2520

ccatatactt tgctccatcg ttcatcggag gaagggttga ctttcaaggg gatcccgaac 2580

agtatctcta tctaa 2595

<210> 5
<211> 28
<212> DNA
<213> Artificial Sequence

<220>
<223> forward primer for K1

<400> 5
catatgatgt ttggcatctt cgacaagg 28

<210> 6
<211> 34
<212> DNA
<213> Artificial Sequence

<220>
<223> reverse primer for K1

<400> 6
ctcgagttag atagagatac tgttcgggat cccc 34

<210> 7
<211> 28
<212> DNA
<213> Artificial Sequence

<220>
<223> forward primer for K1

<400> 7
catatgatga caggtgggat gtttggac 28

<210> 8
<211> 37
<212> DNA
<213> Artificial Sequence

<220>
<223> reverse primer for K2

<400> 8
ctcgagttag atggaaatac tgttgggaat tcctttg 37

## Description

### [Technical Field]

The present invention relates to a method for producing mono- or di-hydroxy derivatives of polyunsaturated fatty acids, and to a use of a complex composition containing derivatives of polyunsaturated fatty acids to alleviate skin wrinkles, alleviate skin inflammation, or strengthen skin barrier.

### [Background Art]

Since an inflammatory response plays an important role as a body's primary defense against pathogen infection and various toxic substances, but at the same time, can act as an underlying factor causing various chronic diseases, the inflammatory response is a physiological phenomenon that needs to be strictly controlled by a sophisticated regulatory mechanism *in vivo.*

Prostaglandin or leukotriene, a derivative of arachidonic acid (C20: 4n-6), which corresponds to an omega-6 polyunsaturated fatty acid, is a representative inflammatory response initiation signal substance and induces an imbalance in the inflammatory response capable of causing various chronic diseases *in vivo.* In general, the inflammatory response is recognized to be naturally terminated when the inflammatory response initiation signal material is not produced, and various steroidal or non-steroidal anti-inflammatory therapeutic agents have been developed to inhibit the production of the substance. However, in recent years, the termination mechanism of the inflammatory response is also performed through a sophisticated regulatory process, and in the process, it was identified that hydroxy derivatives of omega-3 polyunsaturated fatty acids, such as docosahexaenoic acid (DHA, C22: 6n-3) and eicosapentaenoic acid (EPA, C20: 5n-3) may serve as a signaling substance acting in the termination mechanism of the inflammatory response. Those hydroxy derivatives that act as the signal substance for the termination mechanism of the inflammatory response were referred to as specialized proresolving mediators (SPMs; resolvins, protectins, maresins). It was identified that these hydroxy derivatives exhibit an effect superior to existing anti-inflammatory therapeutic agents such as corticosteroids and non-steroid anti-inflammatory drug (NSAID), aspirin.

In addition, unlike conventional steroidal or non-steroidal anti-inflammatory therapeutic agents, which mainly induce inhibition of the production of the inflammatory response initiation signal substance, the SPM has an advantage of showing the efficacy over all stages of the inflammatory response. Thus, research is actively underway on the presence or absence of therapeutic efficacy using the SPM, with respect to various diseases, such as various chronic inflammatory diseases (various chronic inflammatory diseases such as vascular, myocardial infarction, stroke, dementia, osteoarthritis, lung diseases comprising asthma, gastroenteritis, periodontitis, dry eye syndrome, and fatty liver), infection sepsis, burns, wound recovery, pain, cancer, and diabetes.

On the other hand, the SPM, an inflammatory response termination signal substance *in vivo,* has a structure in which two or three hydroxyl groups are inserted into omega-3 polyunsaturated fatty acids, and it is known that the SPM is produced in a very trace amount through a continuous action of two or more enzymes. Therefore, in order to produce SPM sufficient for developing the efficacy and the therapeutic agent of the inflammatory response termination signal substance, efforts to be produced through an organic synthesis process are continued (see Non-Patent Document 1), but there is a limitation that the process thereof is very complicated.

Accordingly, there is a continuous need for the development of a technology capable of efficiently producing mono- or di-hydroxy derivatives using omega-3 polyunsaturated fatty acids through an *in vitro* enzymatic reaction.

On the other hand, the skin is an important organ that protects the human body from external harmful environment and an immune organ with the largest area. The stratum corneum, which constitutes the outermost layer of the skin, has resistance to physical and chemical damage and prevents microorganisms and harmful chemicals from penetrating into the body. In addition, keratinocytes, Langerhans cells, fibroblasts, mast cells, dendritic cells, and capillary endothelial cells form a complex immune system to protect the human body through immune responses when antigens penetrate from the outside.

The skin is constantly being stimulated from the outside (ultraviolet rays, chemicals, heat, physical friction), and recently, new irritants such as fine dust and blue light are increasing. In addition, internal factors such as mental stress, sleep disorders, nutritional imbalance, and drug treatment are known to cause stress on the skin.

According to a recent study, inflammatory response occurs due to skin irritation, and research results are being published that this inflammatory response leads to skin aging. Franceschi first used the term inflammaging in 2000 to explain the correlation between inflammation and aging, and research results are continuously being published that chronic inflammation is the cause of aging or aging-related diseases such as diabetes, arteriosclerosis, and dementia.

Pro-inflammatory cytokines secreted during the initiation of inflammation promote the expression of matrix metalloproteinases (MMPs). It is known that the increase of MMPs is directly related to skin aging, and it promotes aging such as generation of wrinkles and loss of elasticity by decomposing collagen, which accounts for 70% of the skin's constituents.

In general, substances used to inhibit skin aging include enzymes such as superoxide dismutase for removing active oxygen, antioxidants such as vitamin C and tocopherol, botulinum-related peptides, and vitamin A derivatives such as retinol. Among them, vitamin A derivatives have excellent collagen synthesis ability and are the most effective in removing wrinkles, but they have side effects such as instability to light or heat, causing skin irritation, and unstable content in the formulation.

Atopic dermatitis is a disease caused by abnormal genetic, environmental, and immunological reactions and a breakdown of skin barrier, and is a chronic inflammatory skin disease that causes dryness, pruritus and eczema erythematosus. Recently, as prevalence is increasing worldwide due to environmental changes, eating habits, and environmental pollution, interest in solutions is also increasing. However, since the cause is unknown, the underlying treatment is difficult, and most are dependent on symptomatic treatment.

On the other hand, it was identified that hydroxylated derivatives of docosahexaenoic acid (DHA, C22: 6n-3) and eicosapentaenoic acid (EPA, C20: 5n-3) could act as signaling substances acting on the termination mechanism of the inflammatory response, and those acting as signaling substances of the termination mechanism of the inflammatory response were named SPM (specialized proresolving mediators; resolvins, protectins, maresins).

Recently, although research has been actively conducted as a treatment for various chronic inflammatory diseases (for example, blood vessels, myocardial infarction, stroke, dementia, osteoarthritis, asthma and other lung diseases, gastroenteritis, periodontitis, dry eye syndrome, fatty liver, etc.), infection sepsis, burns, wound recovery, pain, cancer, and diabetes using the SPM, there are insufficient studies and experiments on the effects of anti-skin aging, skin barrier strengthening, and atopic dermatitis.

### [Detailed Description of the Invention]

### [Technical Problem]

An aspect of the present invention is directed to providing an enzyme capable of producing mono- or di-hydroxy derivatives from polyunsaturated fatty acids and a method capable of producing mono- or di-hydroxy derivatives of polyunsaturated fatty acids *in vivo* or *in vitro* using the same.

Another aspect of the present invention is directed to providing a cosmetic composition, or a food composition for improving skin condition through skin wrinkle prevention or alleviation, skin aging prevention or alleviation, skin inflammation prevention or alleviation, skin regeneration and skin barrier strengthening.

Yet another aspect of the present invention is directed to providing a pharmaceutical composition for preventing or treating one or more kinds of skin diseases selected from the group consisting of skin wounds, skin scars, dermatitis, atopic dermatitis, pruritus, eczematous skin disease, dry eczema, athlete's foot, erythema, urticaria, psoriasis, weak rash, acne and hair loss.

Yet another aspect of the present invention is directed to providing a method for improving skin condition, wherein the method includes administering an effective amount of a complex composition containing hydroxy derivatives of polyunsaturated fatty acids as an active ingredient to a subject.

Yet another aspect of the present invention is directed to providing a method for preventing or treating a skin disease, wherein the method includes administering a complex composition containing hydroxy derivatives of polyunsaturated fatty acids as an active ingredient to a subject.

Yet another aspect of the present invention is directed to providing a use of a complex composition containing hydroxy derivatives of polyunsaturated fatty acids as an active ingredient in the production of a medicament for preventing or treating a skin disease.

### [Technical Solution]

An aspect of the present invention provides an enzyme for producing mono- or di-hydroxy derivatives of polyunsaturated fatty acids having an amino acid sequence having at least 90% homology with SEQ ID NO: 1 or 2.

Another aspect of the present invention provides a method for producing mono- or di-hydroxy derivatives of polyunsaturated fatty acids *in vitro,* wherein the method includes reacting the enzyme for producing the mono- or di-hydroxy derivatives with polyunsaturated fatty acids.

Yet another aspect of the present invention provides a method for producing mono- or di-hydroxy derivatives of polyunsaturated fatty acids *in vivo,* wherein the method includes culturing a transformant, in which a gene encoding the enzyme for producing the mono- or di-hydroxy derivatives is transduced, in the presence of polyunsaturated fatty acids, and isolating mono- or di-hydroxy derivatives of polyunsaturated fatty acids from the cultured culture.

Yet another aspect of the present invention provides a cosmetic composition for improving skin condition, wherein the cosmetic composition contains 17-hydroxydocosahexaenoic acid (17-HDHA), Resolvin D5, and Protectin DX as active ingredients.

Yet another aspect of the present invention provides a food composition for improving skin condition, wherein the food composition contains 17-hydroxydocosahexaenoic acid (17-HDHA), Resolvin D5, and Protectin DX as active ingredients.

Yet another aspect of the present invention provides a pharmaceutical composition for preventing or treating a skin disease, wherein the pharmaceutical composition contains 17-hydroxydocosahexaenoic acid (17-HDHA), Resolvin D5, and Protectin DX as active ingredients.

Yet another aspect of the present invention provides a method for improving skin condition, wherein the method includes administering an effective amount of a complex composition containing 17-hydroxydocosahexaenoic acid (17-HDHA), Resolvin D5, and Protectin DX as active ingredients to a subject in need of improvement of the skin condition.

Yet another aspect of the present invention provides a method for preventing or treating a skin disease, wherein the method includes administering a complex composition containing 17-hydroxydocosahexaenoic acid (17-HDHA), Resolvin D5, and Protectin DX as active ingredients to a subject in need of treatment of the skin disease.

Yet another aspect of the present invention provides a complex composition containing 17-hydroxydocosahexaenoic acid (17-HDHA), Resolvin D5, and Protectin DX as active ingredients for use in the prevention or treatment of skin diseases.

### [Advantageous Effects]

According to the present invention, the enzyme can produce mono- or di-hydroxy derivatives in a single reaction by using polyunsaturated fatty acids as a substrate and can be very effectively used for *ex vivo* production of mono- or di-hydroxy derivatives of polyunsaturated fatty acids.

The SPMs complex composition containing docosahexaenoic acid hydroxy derivatives of the present invention increases collagen synthesis, restores damaged skin barrier, and reduces transdermal moisture loss, thereby inhibiting skin aging or alleviating skin wrinkles.

In addition, the SPMs complex composition of the present invention has significantly improved the effect of inhibiting the expression of TNF-α, IL-6, the effect of increasing the expression of filaggrin and loricrin, and the antioxidant effect, thereby strengthening the skin barrier, and being effective in preventing or treating various skin diseases comprising atopic dermatitis.

However, the effects of the present invention are not limited to those mentioned above, and other effects not mentioned herein will be clearly understood by those skilled in the art from the following description.

### [Brief Description of Drawings]

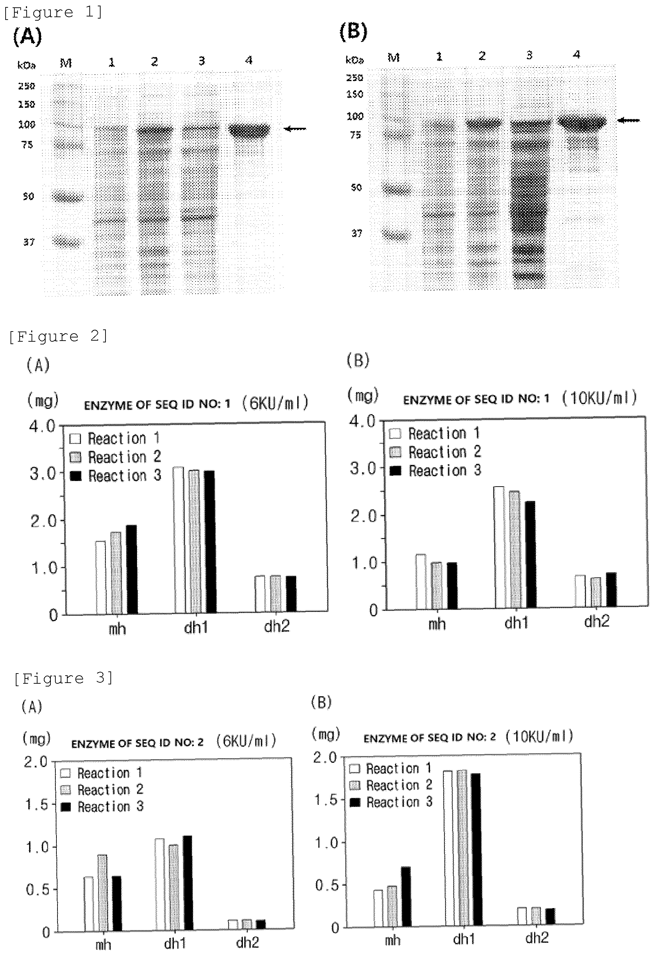

FIG. 1 illustrates results of identifying the overexpression of a protein (A) having an amino acid sequence represented by SEQ ID NO: 1 and a protein (B) having an amino acid sequence represented by SEQ ID NO: 2 by SDS-PAGE.
FIG. 2 illustrates results of performing normal phase HPLC analysis on a reaction product obtained by reacting a protein having an amino acid sequence represented by SEQ ID NO: 1 with docosahexaenoic acid (DHA).
FIG. 3 illustrates results of performing normal phase HPLC analysis on a reaction product obtained by reacting a protein having an amino acid sequence represented by SEQ ID NO: 2 with docosahexaenoic acid (DHA).

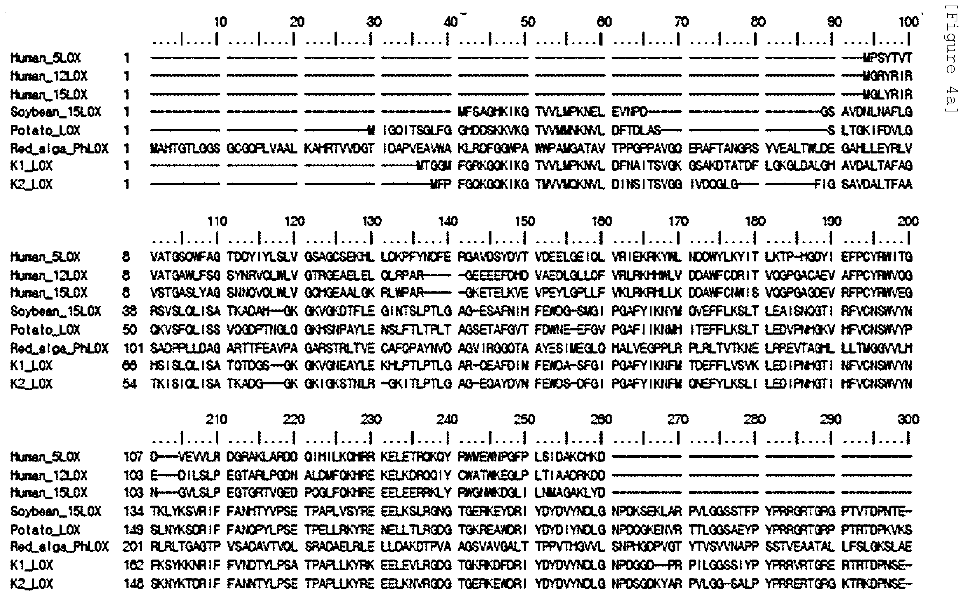

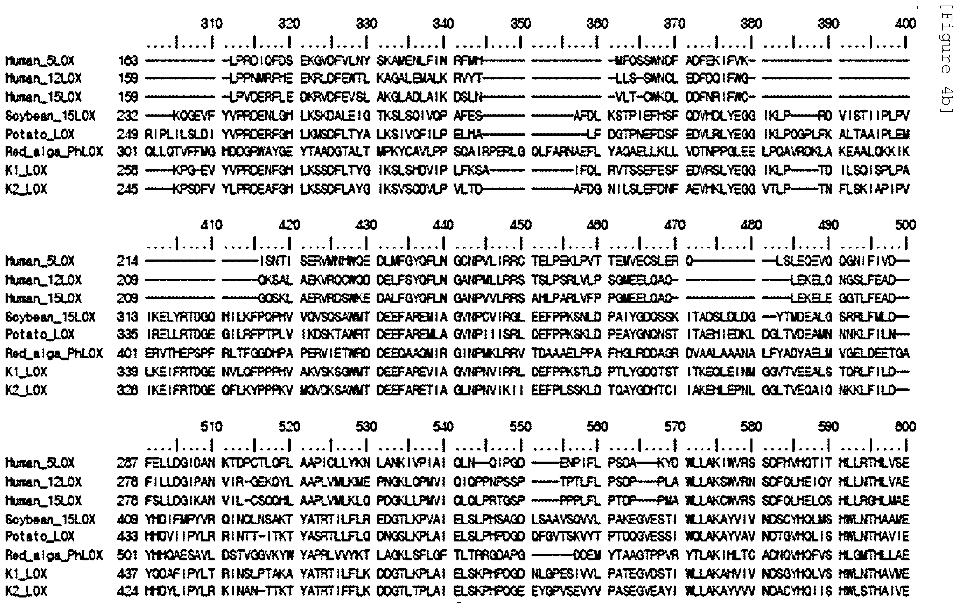

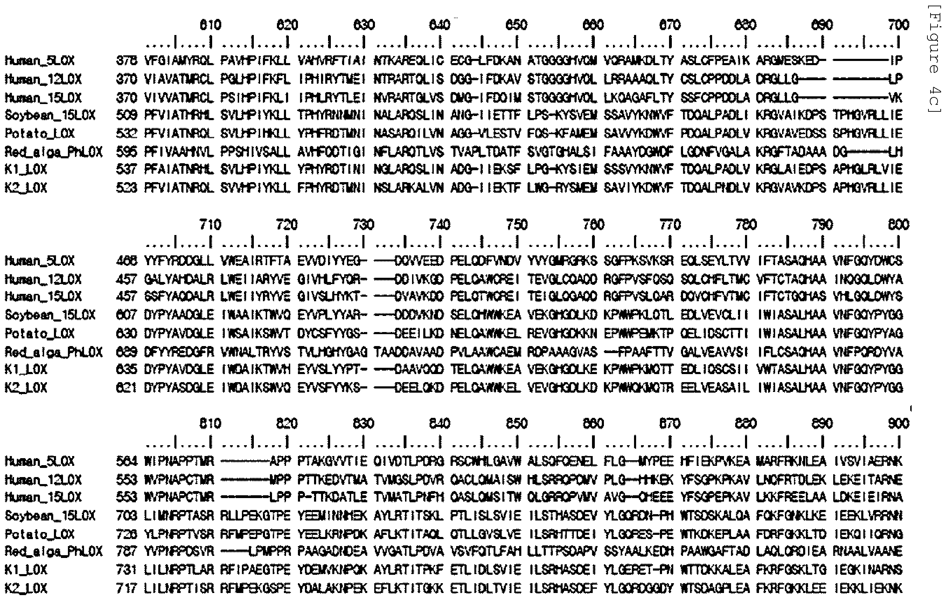

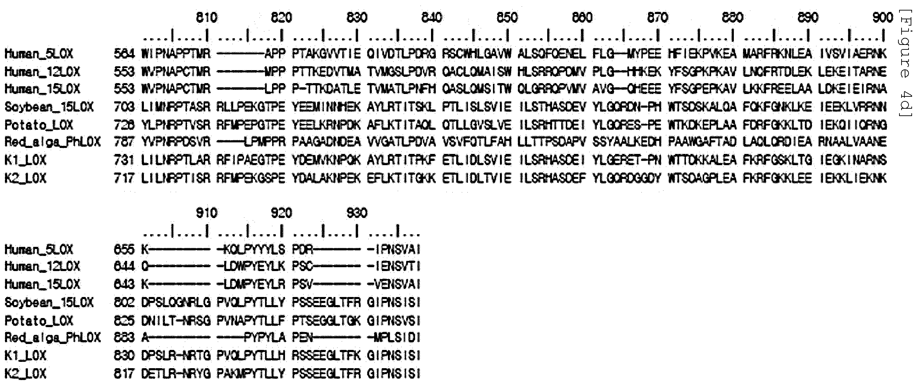

FIGS. 4A to 4D illustrate results of analyzing homology of amino acid sequences with human 5LOX (AAA36183), human 12LOX (AAA51533), human 15LOX (AAA36183), soybean 15LOX (AAA33986), potato LOX (AAB81595), and red algae PhLOX2 (AGN54275), respectively, which are lipoxygenase enzymes with similar activity, with respect to a protein (K1) having an amino acid sequence represented by SEQ ID NO: 1 and a protein (K2) having an amino acid sequence represented by SEQ ID NO: 2.

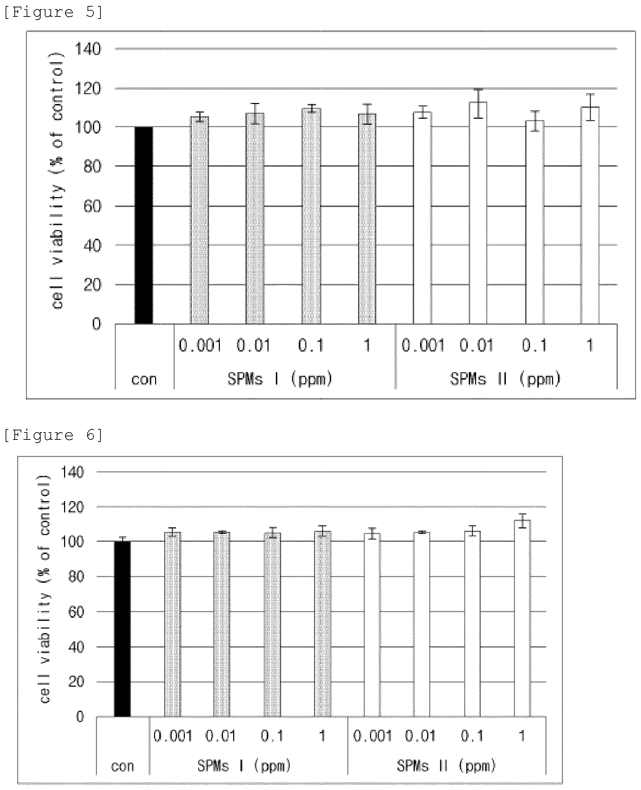

FIG. 5 illustrates a result of identifying the cytotoxicity of a hydroxy derivative produced by a protein having an amino acid sequence represented by SEQ ID NO: 1 (SPM 1) and a hydroxy derivative produced by a protein having an amino acid sequence represented by SEQ ID NO: 2 (SPM 2), with respect to human dermal fibroblasts.
FIG. 6 illustrates a result of identifying the cytotoxicity of a hydroxy derivative produced by a protein having an amino acid sequence represented by SEQ ID NO: 1 (SPM 1) and a hydroxy derivative produced by a protein having an amino acid sequence represented by SEQ ID NO: 2 (SPM 2), with respect to human keratinocytes.

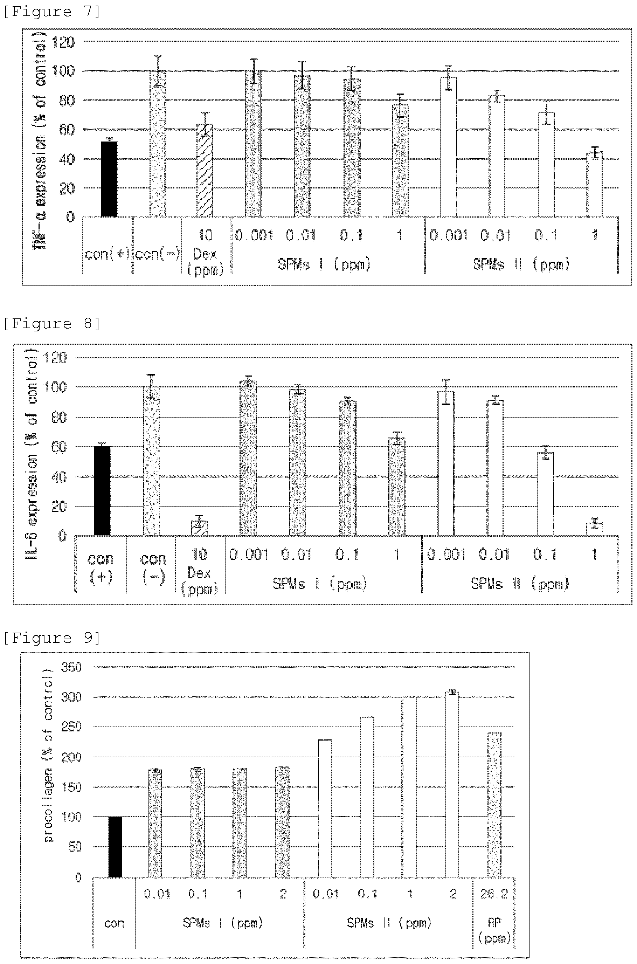

FIGS. 7 and 8 illustrate results of identifying the TNF-α expression inhibitory activity (FIG. 7) and IL-6 expression inhibitory activity (FIG. 8) of a hydroxy derivative produced by a protein having an amino acid sequence represented by SEQ ID NO: 1 (SPM 1) and a hydroxy derivative produced by a protein having an amino acid sequence represented by SEQ ID NO: 2 (SPM 2), with respect to human keratinocytes.
FIG. 9 illustrates a result of identifying the collagen production enhancing activity of a hydroxy derivative produced by a protein having an amino acid sequence represented by SEQ ID NO: 1 (SPM 1) and a hydroxy derivative produced by a protein having an amino acid sequence represented by SEQ ID NO: 2 (SPM 2), with respect to human dermal fibroblasts.

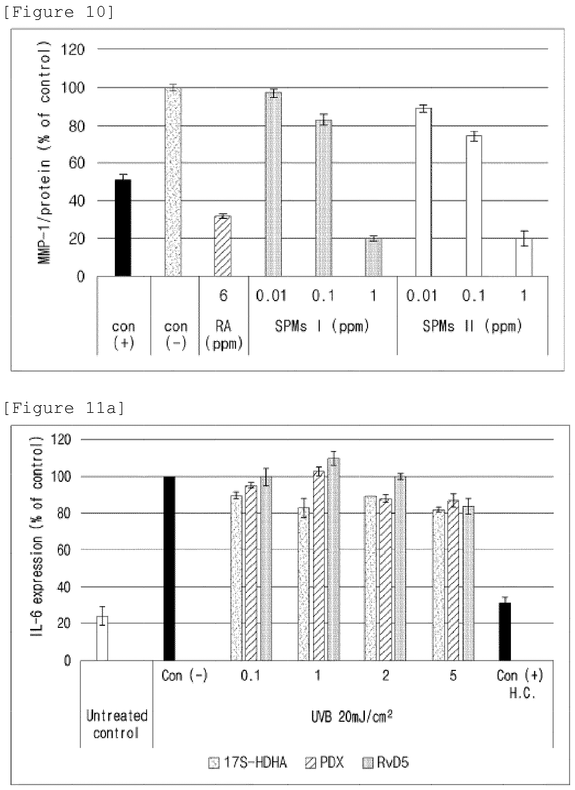

FIG. 10 illustrates a result of identifying the collagenase production inhibitory activity of a hydroxy derivative produced by a protein having an amino acid sequence represented by SEQ ID NO: 1 (SPM 1) and a hydroxy derivative produced by a protein having an amino acid sequence represented by SEQ ID NO: 2 (SPM 2), with respect to human dermal fibroblasts.

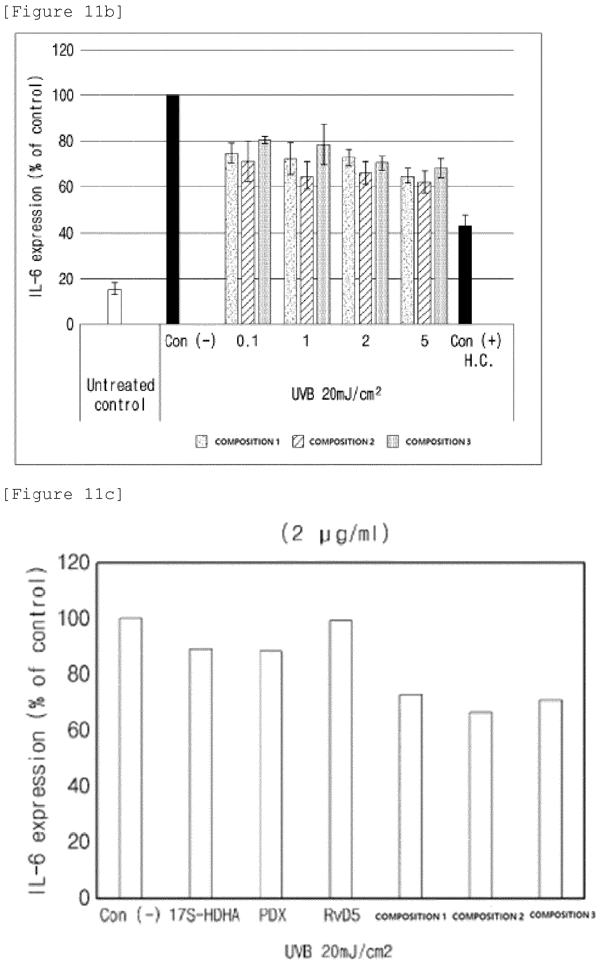

FIG. 11 illustrates a result of comparing the IL-6 expression inhibitory activity of a single component and an SPMs complex composition with respect to human keratinocytes: FIG. 11A illustrates the IL-6 expression inhibitory activity by concentration of each of 17S-HDHA, Protectin DX and Resolvin D5; FIG. 11B illustrates the IL-6 expression inhibitory activity by concentration of the SPMs complex compositions 1 to 3 of the present invention; and FIG. 11C illustrates the synergistic IL-6 expression inhibitory effect of the SPMs complex compositions of the present invention compared to the single component [Untreated control: group not irradiated with UVB, Con(-): group not treated with anything after irradiating UVB, Con(+) H.C.: positive control group treated with hydrocortisone after UVB irradiation].

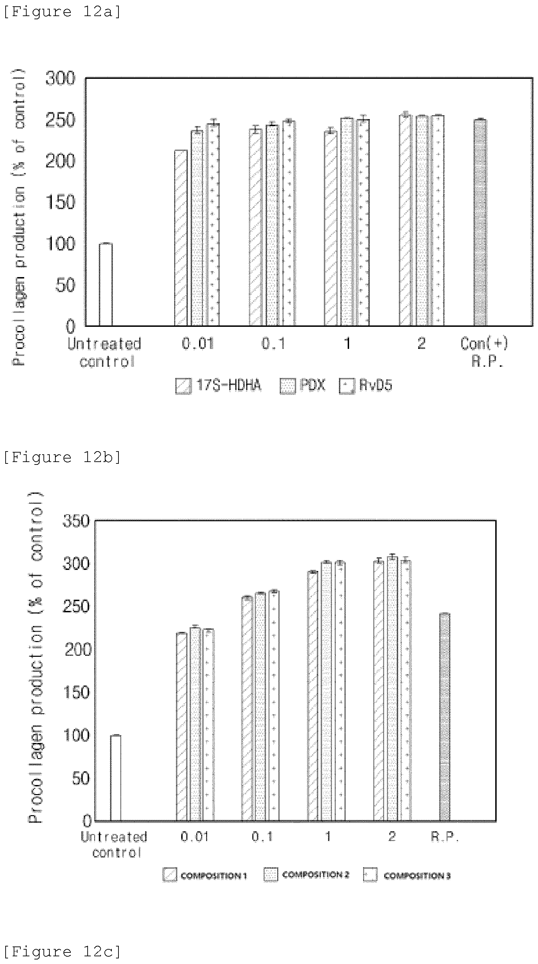

FIG. 12 illustrates a result of identifying the procollagen synthesis effect of a single component and an SPMs complex composition with respect to human dermal fibroblasts: FIG. 12A illustrates the procollagen synthesis effect by concentration of each of 17S-HDHA, Protectin DX and Resolvin D5; FIG. 12B illustrates the procollagen synthesis effect by concentration of the SPMS complex compositions 1 to 3 of the present invention; and FIG. 12C illustrates the synergistic procollagen synthesis effect of the SPMs complex composition of the present invention compared to the SPM single component [Untreated control: untreated group, Con(+) R.P.: positive control group treated with retinyl palmitate].

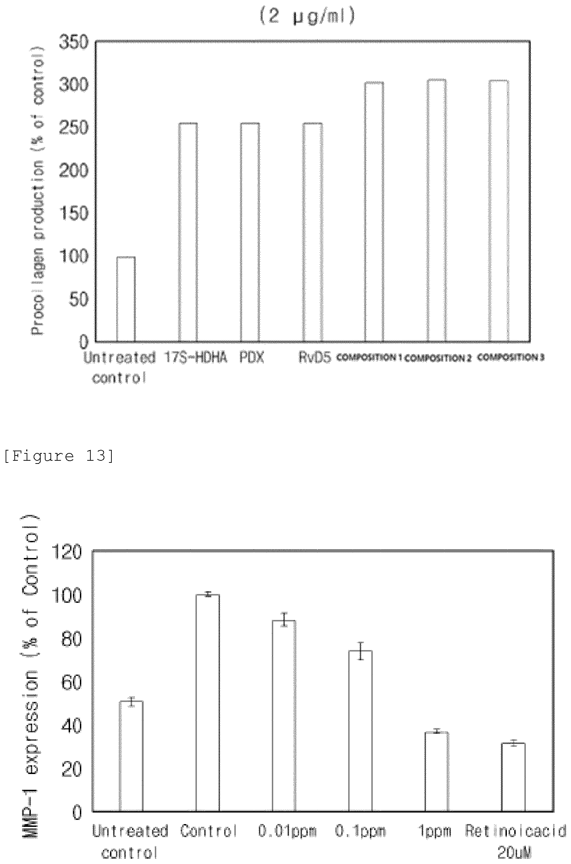

FIG. 13 illustrates a result of identifying the effect of improving the collagenase (MMP-1) expression inhibitory ability of the SPMs complex composition of the present invention with respect to human dermal fibroblasts [Untreated control: group not irradiated with UVB, Control: group not treated with anything after irradiating UVB, Retinoic acid: positive control group treated with retinoic acid after UVB irradiation].

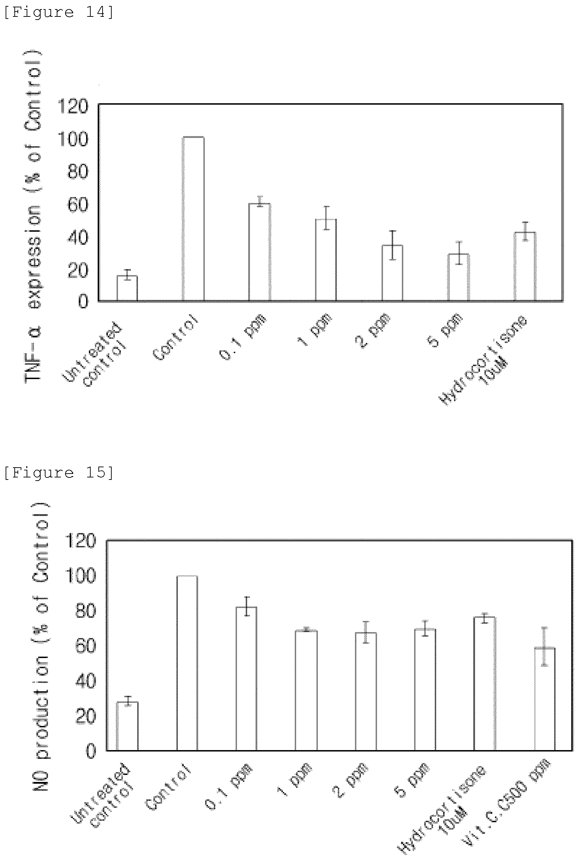

FIG. 14 illustrates a result of identifying the effect of improving the TNF-α expression inhibitory ability of the SPMs complex composition of the present invention with respect to human keratinocytes [Untreated control: group not irradiated with UVB, Control: group not treated with anything after irradiating UVB, Hydrocortisone: positive control group treated with hydrocortisone after UVB irradiation].
FIG. 15 illustrates a result of identifying the NO production reduction effect of the SPMs complex composition of the present invention [Untreated control: group not irradiated with UVB, Control: group not treated with anything after irradiating UVB, Hydrocortisone: positive control group treated with hydrocortisone after UVB irradiation, Vit.C: positive control group treated with vitamin C after UVB irradiation].

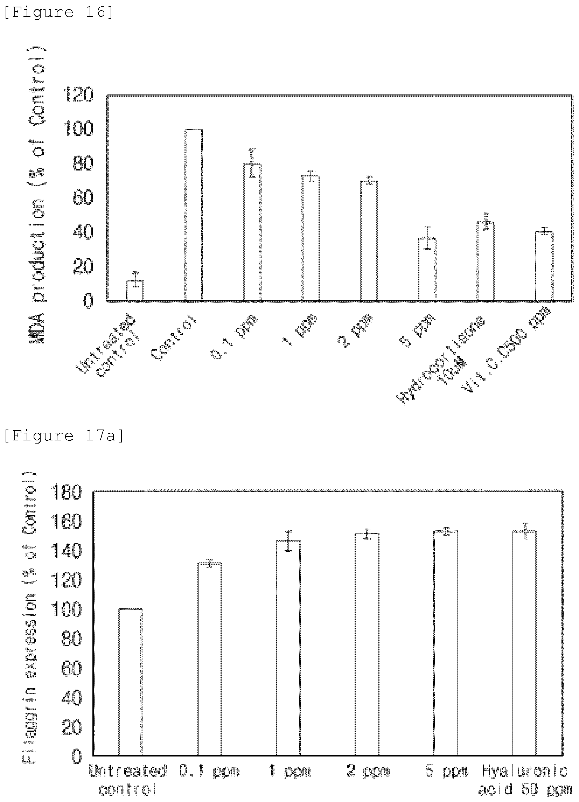

FIG. 16 illustrates a result of identifying the effect of reducing the lipid peroxide production of the SPMs complex composition of the present invention with respect to the stimulation induced by fine dust [Untreated control: group not treated with fine dust, Control: group not treated with anything after inducing irritation with fine dust, Hydrocortisone: positive control group treated with hydrocortisone after inducing irradiation with fine dust, Vit.C: positive control group treated with vitamin C after inducing irradiation with fine dust].

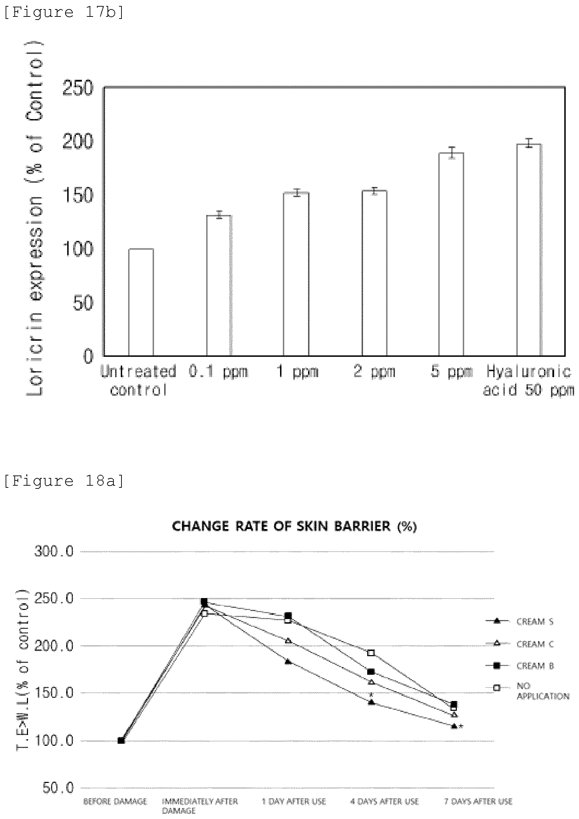

FIG. 17 illustrates a result of identifying the skin barrier improvement effect of the SPMs complex composition of the present invention through the expression level of the core protein constituting the skin barrier: FIG. 17A illustrates a result of identifying the increase in the expression of filaggrin, and FIG. 17B illustrates a result identifying the increase in the expression of loricrin [untreated control: group not treated with anything, Hyaluronic acid: positive control group treated with hyaluronic acid].

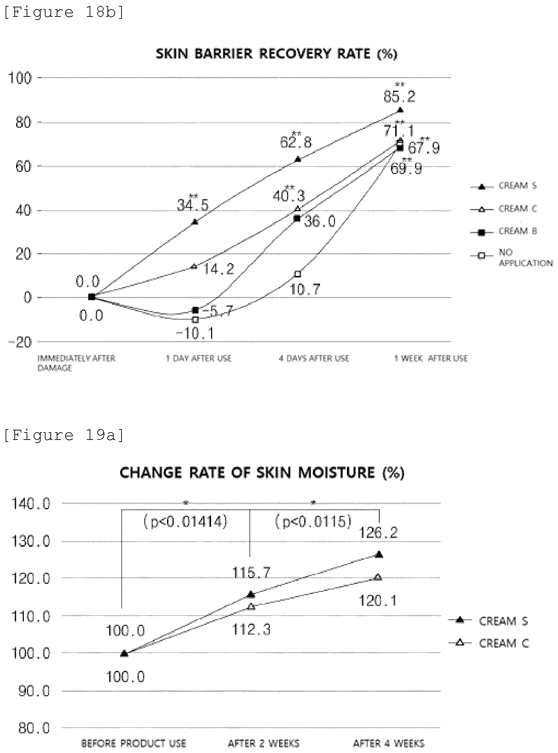

FIG. 18 illustrates a result of identifying the skin barrier improvement effect by the SMPs complex composition of the present invention through human efficacy evaluation: FIG. 18A illustrates the change rate of the skin barrier index (%), and FIG. 18B illustrates the skin barrier recovery rate (%).

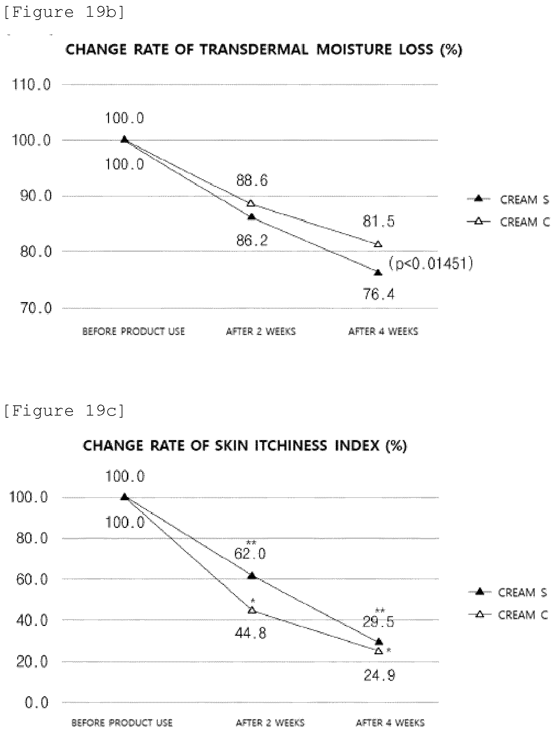

FIG. 19 illustrates a result of identifying the improvement effect of atopic dermatitis by the SMPs complex composition of the present invention through human efficacy evaluation: FIG. 19A illustrates a change rate of skin moisture (%) at the lesion site, FIG. 19B illustrates a change rate of transdermal moisture loss (%) at the lesion site, and FIG. 19C illustrates a change rate of skin itchiness index (5) at the lesion site.

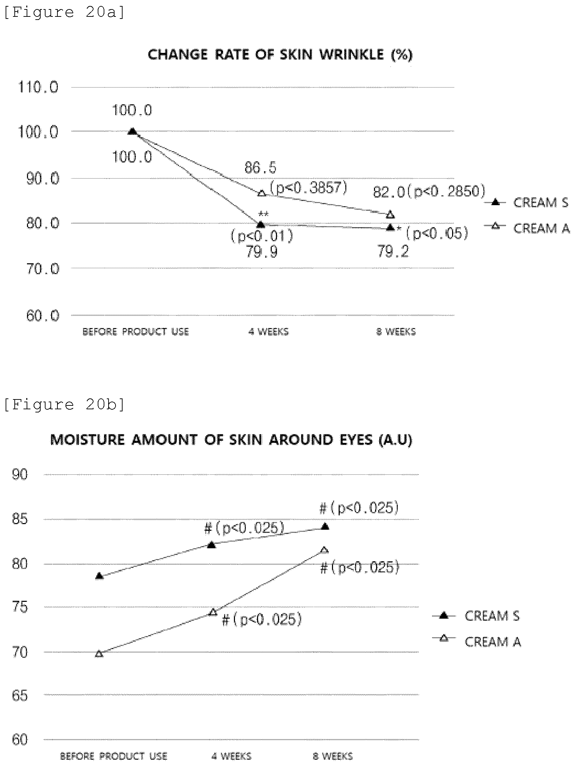

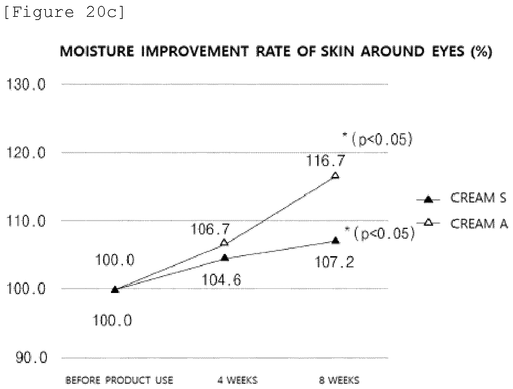

FIG. 20 illustrates a result of identifying the effect of alleviating eye wrinkles and improving skin moisture by the SPMs complex composition of the present invention through human efficacy evaluation: FIG. 20A illustrates a change rate of eye wrinkles (%), FIG. 20B illustrates a moisture amount of the skin around the eyes, and FIG. 20C illustrates a moisture improvement rate of the skin around the eyes (%).

### [Best Mode]

Hereinafter, the present invention will be described in detail.

### 1. Enzyme for Producing Mono- or Di-Hydroxy Derivatives of Polyunsaturated Fatty Acids

An aspect of the present invention provides an enzyme for producing or mono- or di-hydroxy derivatives of polyunsaturated fatty acids.

In addition, another aspect of the present invention provides a nucleic acid molecule encoding the enzyme for producing the mono- or di-hydroxy derivatives of polyunsaturated fatty acids.

The enzyme for producing the mono- or di-hydroxy derivatives of polyunsaturated fatty acids of the present invention includes an amino acid sequence represented by SEQ ID NO: 1 or 2, and may be variants or fragments of amino acids having different sequences by deletion, insertion, substitution, or a combination of amino acid residues, within a range that does not affect the function of the enzyme for producing the mono- or di-hydroxy derivatives of polyunsaturated fatty acids. Amino acid exchange has been known in the pertinent field at protein and peptide levels without changing entirely the activity of the enzyme for producing the mono- or di-hydroxy derivatives of polyunsaturated fatty acids. In some cases, the amino acid exchange may be modified by phosphorylation, sulfation, acrylation, glycosylation, methylation, farnesylation, and the like. Accordingly, the present invention includes a protein having an amino acid sequence substantially identical to the protein comprising the amino acid sequence represented by SEQ ID NO: 1 or 2, and variants or active fragments thereof. The substantially identical proteins mean proteins having homology with the amino acid sequence of at least 90%, preferably at least 93%, and most preferably at least 95%, but are not limited thereto, and the proteins having homology of at least 90% with the amino acid sequence and identical enzyme activity are included in the scope of the present invention.

The gene of the enzyme for producing the mono- or di-hydroxy derivative of polyunsaturated fatty acids preferably consists of a nucleotide sequence represented by SEQ ID NO: 3 or 4. However, the nucleic acid molecules encoding the enzyme for producing the mono- or di-hydroxy derivatives of polyunsaturated fatty acids of the present invention, and variants or active fragments thereof may have various modifications made in an encoding region within a range without changing the amino acid sequence of the enzyme expressed from the encoding region and the variant or active fragment thereof. Various mutations may be made within a range without affecting the expression of the gene even in portions other than the encoding region, and such mutant genes are also included in the scope of the present invention. Accordingly, the present invention includes a gene consisting of a nucleotide sequence substantially identical to the nucleic acid molecule of SEQ ID NO: 3 or 4 and fragments of the gene. The genes consisting of the substantially identical nucleotide sequences mean genes having sequence homology of 80% or more, preferably 90% or more, most preferably 95% or more, but are not limited thereto, and genes having sequence homology of 80% or more and having the identical enzyme activity of the encoded protein are included in the present invention. As such, the gene of the enzyme for producing the mono- or di-hydroxy derivatives of polyunsaturated fatty acids of the present invention may be mutated by substitution, deletion, and insertion of at least one nucleotide, or a combination thereof, as long as the gene encodes a protein having equivalent activity thereto, and these mutants are also included in the scope of the present invention.

The amino acid sequence represented by SEQ ID NO: 1 included in the enzyme for producing the mono- or di-hydroxy derivatives of polyunsaturated fatty acids is preferably encoded by a gene consisting of the nucleotide sequence represented by SEQ ID NO: 3, and the amino acid sequence represented by SEQ ID NO: 2 is preferably encoded by a gene consisting of the nucleotide sequence represented by SEQ ID NO: 4, but the present invention is not limited thereto. The amino acid sequence may also be encoded by a gene consisting of another nucleotide sequence substantially identical to the nucleotide sequence represented by SEQ ID NO: 3 or 4 as long as the amino acid sequence may encode the protein of the present invention having the identical amino acid sequence. These nucleotide sequences may be single-stranded or double-stranded, and may be DNA molecules or RNA molecules.

In a specific example of the present invention, the present inventors had isolated and purified the protein having the amino acid sequence represented by SEQ ID NO: 1 or 2 to find the functions of the protein having the amino acid sequence represented by SEQ ID NO: 1 or 2 (see FIGS. 1). As a result of studying the activities thereof, the present inventors identified that the protein having the amino acid sequence represented by SEQ ID NO: 1 or 2 had the activity of an enzyme capable of producing the mono- or di-hydroxy derivatives with two hydroxyl groups introduced into polyunsaturated fatty acids (see FIGS. 2 and 3).

### 2. Expression Vector and Transformant of Enzyme for Producing Mono- or Di-Hydroxy Derivatives of Polyunsaturated Fatty Acids

Another aspect of the present invention provides a recombinant expression vector and a transformant introduced with the expression vector comprising a gene of the enzyme for producing the mono- or di-hydroxy derivatives of polyunsaturated fatty acids of the present invention.

The recombinant expression vector of the present invention includes the gene of the enzyme for producing the mono- or di-hydroxy derivatives of polyunsaturated fatty acids.

The expression vector includes a plasmid vector, a cosmid vector, a bacteriophage vector, a viral vector, and the like, but is not limited thereto.

The recombinant expression vector may combine expression regulatory sequences such as promoters, terminators, enhancers, etc., or sequences for secretion appropriately depending on the purpose according to a type of host cell to produce the enzyme for producing the mono- or di-hydroxy derivatives of polyunsaturated fatty acids of the present invention.

The expression vector may further include a selection marker for selecting a host cell into which the vector has been introduced, and may include an origin of replication in the case of a replicable expression vector.

In addition, the recombinant expression vector may include a sequence for facilitating purification of the expressed protein, and specifically, may be linked with a gene encoding a tag for isolation and purification so as to be operable to the gene encoding the enzyme for producing the mono- or di-hydroxy derivatives of polyunsaturated fatty acids of the present invention. At this time, the tag for isolation and purification may use GST, poly-Arg, FLAG, histidine-tag (His-tag), c-myc, or the like alone or may also use by sequentially linking two or more of the tags.

The gene encoding the enzyme for producing the mono- or di-hydroxy derivatives of polyunsaturated fatty acids may be cloned through a restriction enzyme cleavage site. When a gene encoding a protein cleavage enzyme recognition site is used in the vector, the gene is linked with the gene of the enzyme for producing the mono- or di-hydroxy derivatives of polyunsaturated fatty acids in frame. When the enzyme is obtained and then cleaved by the protein cleavage enzyme, an original type enzyme for producing the mono- or di-hydroxy derivatives of polyunsaturated fatty acids may be produced.

In a specific example of the present invention, the gene (the gene comprising the nucleotide sequence represented by SEQ ID NO: 3 or 4) of the enzyme for producing the mono- or di-hydroxy derivatives of polyunsaturated fatty acids of the present invention are inserted into a plasmid vector pET28a(+) to prepare a recombinant cloning vector. In addition to the pET28a(+) used in the preparation of the cloning vector, various vectors for prokaryotic cells or eukaryotic cells (such as pPIC and pPICZ, etc.) are known, and thus, various expression vectors other than the vectors may be used depending on the purpose of expression.

The recombinant expression vector of the present invention includes the gene of the enzyme for producing the mono- or di-hydroxy derivatives of polyunsaturated fatty acids, and thus may be effectively used as a vector capable of producing the gene of the enzyme.

In addition, the transformant of the present invention is introduced with the recombinant expression vector comprising the gene encoding the enzyme for producing the mono- or di-hydroxy derivatives of polyunsaturated fatty acids.

The recombinant expression vector according to the present invention is transformed into any one suitable host cell selected from the group consisting of bacteria, yeast, *E. coli,* fungi, plant cells, and animal cells depending on the purpose of expression to prepare a transformant. For example, the host cell may be *E. coli (E. coli* BL21 (DE3), DH5a, etc.), yeast cells *(Saccharomyces* genus, *Pichia* genus, etc.), or the like. At this time, appropriate culturing methods and medium conditions, etc. may be easily selected by those skilled in the art from known techniques in the art depending on a type of host cell.

As a method for introducing the recombinant expression vector for the preparation of the transformant of the present invention, known techniques, that is, a heat shock method, an electric shock method, and the like may be used.

In a specific example of the present invention, the transformant was prepared by transforming, into *E. coli,* the recombinant expression vector comprising the gene encoding the enzyme for producing the mono- or di-hydroxy derivatives of polyunsaturated fatty acids of the present invention by using *E. coli* as a host cell.

Since the protein expressed from the transformant is a protein of a novel sequence having the activity of the enzyme for producing the mono- or di-hydroxy derivatives of polyunsaturated fatty acids, the transformant is mass-cultured to express the gene, thereby facilitating the mass-production of the enzyme for producing the mono- or di-hydroxy derivatives of polyunsaturated fatty acids.

### 3. Method for Producing Mono- or Di-Hydroxy Derivatives of Polyunsaturated Fatty Acids In Vitro

Yet another aspect of the present invention provides a method for producing mono- or di-hydroxy derivatives of polyunsaturated fatty acids *in vitro.*

The method for producing the mono- or di-hydroxy derivatives of polyunsaturated fatty acids *in vitro* of the present invention includes reacting polyunsaturated fatty acids with the enzyme for producing the mono- or di-hydroxy derivatives described in the item of "*1. Enzyme for Producing Mono- or Di-hydroxy Derivatives of Polyunsaturated Fatty Acids."* The polyunsaturated fatty acids are fatty acids containing three or more double bonds between carbons, specifically omega-3 fatty acids, more specifically docosahexaenoic acid or eicosapentaenoic acid, but are not limited thereto. As described above, when the polyunsaturated fatty acids are used as a substrate of the enzyme for producing the mono- or di-hydroxy derivatives, the substrate includes multiple double bonds into which hydroxyl groups may be introduced to achieve the production of the mono- or di-hydroxy derivatives.

The method for producing the mono- or di-hydroxy derivatives of polyunsaturated fatty acids *in vitro* of the present invention further includes recovering mono- or di-hydroxy derivatives from a reaction product of the enzyme and the polyunsaturated fatty acids.

The recovering may be achieved by performing a method commonly performed in the art, such as centrifugation and filtration, and may be achieved by additionally performing a purification process in a general manner. For example, the purification process may be performed alone or in combination with techniques, such as solvent precipitation, dialysis, gel filtration, ion exchange, and chromatography such as reverse phase column chromatography.

The reacting of the polyunsaturated fatty acids with the enzyme for producing the mono- or di-hydroxy derivatives may be performed in a temperature range of 10°C to 40°C, preferably in a temperature range of 15°C to 35°C, but is not limited thereto. In addition, the reacting may be performed in a range of pH 4 to pH 10, preferably in a range of pH 7 to pH 9, but is not limited thereto. When the reacting is performed in the range of the temperature and pH, the activity of the enzyme for producing the mono- or di-hydroxy derivatives is maximized to more effectively produce the mono- or di-hydroxy derivatives.

### 4. Method for Producing Mono- or Di-Hydroxy Derivatives of Polyunsaturated Fatty Acids in vivo

Another aspect of the present invention provides a method for producing mono- or di-hydroxy derivatives of polyunsaturated fatty acids *in vivo.*

The method for producing the mono- or di-hydroxy derivatives of polyunsaturated fatty acids *in vivo* of the present invention includes culturing the transformant described in the item of "*2*. *Expression Vector and Transformant of Enzyme for Producing Mono- or Di-Hydroxy Derivatives of Polyunsaturated Fatty Acids"* in the presence of polyunsaturated fatty acids and isolating mono- or di-hydroxy derivatives of polyunsaturated fatty acids from the cultured culture.

In the transformant introduced with the recombinant expression vector containing the gene encoding the enzyme for producing the mono- or di-hydroxy derivatives, the enzyme for producing the mono- or di-hydroxy derivatives may be expressed. With respect to the enzyme for producing the mono- or di-hydroxy derivatives present in the transformant, a detailed description thereof will be omitted by citing the description of the item of "*1. Enzyme for Producing Mono- or Di-Hydroxy Derivatives of Polyunsaturated Fatty Acids."*

In the transformant, the enzyme for producing the mono- or di-hydroxy derivatives is expressed. As a result, by using the same, the transformant is cultured in a culture medium containing polyunsaturated fatty acids as a substrate to produce the mono- or di-hydroxy derivatives without a separate process of isolating the enzyme.

The culturing of the transformant may be performed according to an appropriate medium and culturing conditions known in the art. Those skilled in the art may easily adjust and use the medium and culturing conditions according to a type of transformant to be selected. The culturing method may include a batch type, a continuous type, a fed-batch type, or a combination thereof.

The medium may contain various carbon sources, nitrogen sources, and trace element ingredients.

The carbon sources may include, for example, carbohydrates such as glucose, sucrose, lactose, fructose, maltose, starch, and cellulose, fats such as soybean oil, sunflower oil, castor oil, and coconut oil, fatty acids such as palmitic acid, stearic acid, and linoleic acid, alcohols such as glycerol and ethanol, organic acids such as acetic acid, or combinations thereof. The culturing may be performed using glucose as the carbon source. The nitrogen sources may include organic nitrogen sources such as peptone, yeast extract, broth, malt extract, corn steep liquor (CSL), and soybean milk, inorganic nitrogen sources such as urea, ammonium sulfate, ammonium chloride, ammonium phosphate, ammonium carbonate, and ammonium nitrate, or combinations thereof. As a supply source of phosphorus, the medium may contain, for example, potassium dihydrogen phosphate, dipotassium hydrogen phosphate and sodium-containing salts corresponding thereto, and metal salts such as magnesium sulfate or iron sulfate.

In addition, amino acids, vitamins, suitable precursors, and the like may be included in the medium. The medium or individual ingredients may be added to the culture medium in a batch or continuous type.

In addition, during the culturing, production of bubbles may be inhibited by using an anti-foaming agent such as fatty acid polyglycol ester.

### Composition for Improving Skin Condition or Preventing or Treating Skin Disease

On the other hand, yet another aspect of the present invention provides a composition for improving skin conditions or preventing or treating skin diseases.

In a specific embodiment, the present invention provides a composition for improving skin condition containing 17-hydroxydocosahexaenoic acid (17-HDHA), Resolvin D5, and Protectin DX as active ingredients.

The 17-hydroxydocosahexaenoic acid (17-HDHA), Resolvin D5, and Protectin DX are lipoxygenase (LOX) metabolite derived from an omega-3 fatty acid, that is, docosahexaenoic acid (DHA), and is a specialized proresolving mediator (SPM).

The 17-hydroxydocosahexaenoic acid (17-HDHA) is (±) 17-hydroxy (dihydroxy)-4Z, 7Z, 10Z, 13Z, 15E, 19Z-docosahexaenoic acid (docosahexaenoic acid), and is represented by the structure of Formula 1 below.

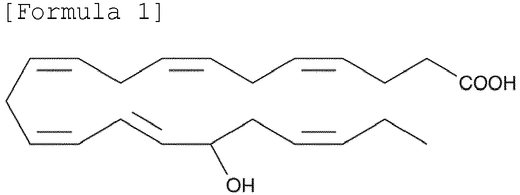

The Resolvin D5 (RvD5) is 7S, 17S-dihydroxy-4Z, 8E, 10Z, 13Z, 15E, 19Z-docosahexaenoic acid (docosahexaenoic acid), and is represented by the structure of Formula 2 below.

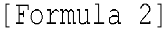

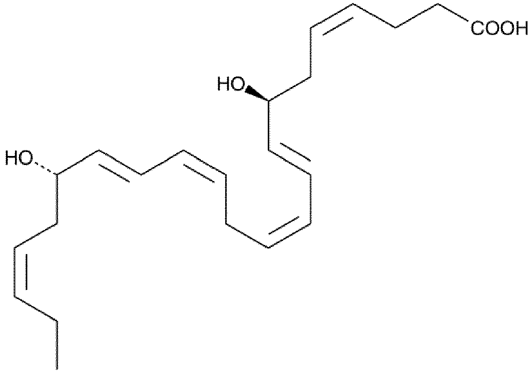

The Protectin DX (PDX) is 10S, 17S-dihydroxy-4Z,7Z,11E,13Z,15E,19Z-docosahexaenoic acid and is an isomer of Protectin D1, and is represented by the structure of Formula 3 below.

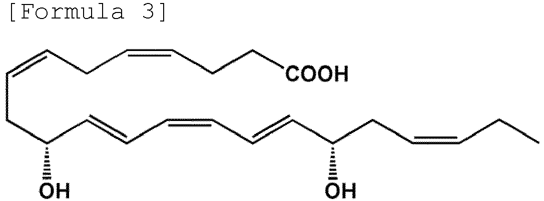

The 17-HDHA, Resolvin D5, and Protectin DX may be biosynthesized by treating the enzyme of SEQ ID NO: 1 or 2 of the present invention to docosahexaenoic acid, chemically synthesized, or commercially purchased, but are not limited thereto.

In a specific example of the present invention, as a result of treating human dermal fibroblasts with a complex composition containing 17-hydroxydocosahexaenoic acid (17-HDHA), Resolvin D5, and Protectin DX, it was identified that the expression of inflammatory factors increased by UV irradiation was suppressed and procollagen synthesis was increased. In particular, it was identified that the complex composition of the present invention synergistically induced the collagen synthesis effect and the inflammation inhibitory effect compared to the single compound. Furthermore, in human application experiments, it was identified that the complex composition of the present invention relieved xeroderma and itching caused by atopic dermatitis by restoring the damaged skin barrier and reducing transdermal moisture loss.

Accordingly, the composition of the present invention may be for improving skin condition.

As used herein, the term "skin condition" may be skin aging due to ultraviolet rays, spots, freckles, skin wounds, dermatitis, atopic dermatitis, pruritus, eczematous skin disease, dry eczema, erythema, urticaria, psoriasis, weak rash or acne.

The skin aging shows symptoms such as reduction in elasticity of the skin, reduction in shine, generation of wrinkles, decreased regeneration, or severe drying, and includes both intrinsic and extrinsic aging caused by the passage of time or external environments. In particular, the skin aging may be photoaging, and the photoaging refers to skin damage that occurs when the skin is repeatedly or long-term exposed to ultraviolet rays.

In addition, the wrinkle means a wrinkle occurring in all body parts comprising the face, and includes both static rhytides and dynamic rhytides.

As used herein, the term "improving skin condition" is a concept that includes all of prevention or alleviation of skin wrinkles, prevention or alleviation of skin aging, prevention or alleviation of skin inflammation, proliferation and regeneration of skin cells, contraction or reduction of pores, improvement of skin barrier function, alleviation of skin irritation, antioxidant, and collagen synthesis enhancement.

As used herein, the meaning of "containing as an active ingredient" is containing an effective amount to the extent that may exhibit a skin improvement effect, for example, wrinkle alleviation, inflammation alleviation, skin regeneration, atopic dermatitis alleviation, or skin barrier strengthening and improvement.

According to one embodiment of the present invention, the composition may promote collagen synthesis in cells or inhibit collagen decomposition by inhibiting the expression or activity of matrix metalloproteinase (MMP). The composition may, for example, inhibit the decomposition of the extracellular matrix by inhibiting the expression or activity of MMP-1 that decomposes type 1 collagen. According to one embodiment of the present invention, the composition may have antioxidant activity. The composition may inhibit the formation of lipid peroxide. According to another embodiment of the present invention, the composition may inhibit an inflammatory factor. The composition may inhibit NO, TNF-α, and IL-6, which are inflammation initiators.

The TNF-α (tumor necrosis factor-a) and IL-6 (Interleukin-6) are representative inflammation-inducing cytokines. TNF-α is a pro-inflammatory cytokine produced by lymphocytes activated by lipopolysaccharide (LPS), an endotoxin in the cell membrane of Gram-negative bacteria, and IL-6 is secreted by macrophages and T cells to stimulate the immune response to promote inflammation.

The complex composition containing 17-HDHA, Resolvin D5, and Protectin DX of the present invention may implement excellent anti-inflammatory action by suppressing the expression of inflammatory cytokines TNF-α and IL-6, and has an excellent antioxidant effect of inhibiting the production of lipid peroxides due to oxidative stress occurring in the skin, thereby improving skin resistance ability to external stimuli.

According to yet another embodiment of the present invention, the composition may be to strengthen the skin barrier.

The stratum corneum, which is the outermost layer of the epidermis of the skin barrier, is mainly composed of non-nucleated flat corneocytes. The multi-lamellar lipid membrane formed of intercellular lipids such as ceramide, cholesterol, and fatty acids synthesized by keratinocytes of the skin barrier maintained through the division and differentiation process of normal epidermal cells acts as a protective barrier to prevent the moisture in the skin from evaporating. Among these intercellular lipids, omega hydroxy ceramide is chemically covalently linked with involucrin, a protein of the outer layer of corneocytes, to form a corneocyte lipid envelope (CLE), so as to play a role in physically stabilizing intercellular lipids in the form of a multi-layered lipid membrane, thereby strengthening the barrier function.

The composition of the present invention is delivered to the stratum corneum through skin application and promotes the differentiation of keratinocytes, thereby producing an effect of increasing the thickness of the epidermal layer. Moreover, the composition has an excellent effect of restoring damage to the skin barrier, and thus may be usefully used for the treatment and prevention of skin diseases caused by damage to the skin barrier. Skin diseases caused by damage to the skin barrier include, but are not limited to, atopic dermatitis, xeroderma, psoriasis, ichthyosis, acne, and the like.

In addition, the skin barrier strengthening effect was identified by increasing the expression of filaggrin and loricrin. Filaggrin is one of several structural proteins expressed by keratinocytes in the differentiation stage and is involved in differentiation from the basal layer of the epidermis to the stratum corneum. It is used as a major indicator of skin moisture retention and skin membrane function as it also forms the main agent of natural moisture factor (NMF) essential for maintaining moisture in the skin tissue. The complex composition containing 17-HDHA, Resolvin D5, and Protectin DX according to the present invention has excellent skin barrier protection, strengthening, and improvement functions as the gene expression of filaggrin or loricrin is significantly increased.

The complex composition containing 17-HDHA, Resolvin D5, and Protectin DX of the present invention may implement a remarkably improved IL-6 expression inhibitory effect and procollagen synthesis effect, particularly in comparison with using each of these compounds at the same concentration due to the complex synergistic action of 17-HDHA, Resolvin D5, and Protectin DX, which are contained as active ingredients (FIGS. 11 and 12).

In one embodiment of the present invention, the complex contains the 17-HDHA, Resolvin D5, and Protectin DX in a weight ratio of 3 to 85 : 10 to 60 : 5 to 50, for example, in a weight ratio of 3 : 10 : 5, in a weight ratio of 5 : 60 : 50, and in a weight ratio of 85 : 10 : 5, but is not limited thereto. In one example of the present invention, the skin improvement effect was identified using a complex composition containing 17-HDHA, Resolvin D5, and Protectin DX in weight ratios of 3 : 47 : 50, 25 : 56 : 19, and 84 : 10 : 6.

On the other hand, the composition for improving skin condition may be used as a cosmetic composition, a pharmaceutical composition, or a food composition.

Accordingly, in one aspect, the present invention provides a cosmetic composition for improving skin condition containing 17-hydroxydocosahexaenoic acid (17-HDHA), Resolvin D5, and Protectin DX as active ingredients.

The complex composition containing the 17-hydroxydocosahexaenoic acid (17-HDHA), Resolvin D5, and Protectin DX, and the content related to skin condition improvement are the same as described above.

When the complex composition of the present invention is used as a cosmetic composition, components commonly used in cosmetic compositions are included in addition to the 17-HDHA, Resolvin D5, and Protectin DX, for example, a sulfating agent, a stabilizer, a solubilizer, a vitamin, customary adjuvants such as pigments and fragrances, and carriers.

The cosmetic composition of the present invention may be prepared as any formulation commonly prepared in the art. The cosmetic composition may be formulated as, for example, a solution, a suspension (anhydrous and aqueous), an anhydrous product (oils and glycols), an emulsion, a paste, a gel, a mask, a pack, a powder, a cream, a lotion, a powder, a soap, a surfactant-containing cleanser, an oil, a powdered foundation, an emulsion foundation, a wax foundation, a spray or the like, but is not limited thereto. More specifically, the cosmetic composition may be prepared as a formulation such as a softening toner (skin), a nutrient toner (milk lotion), a nutrient cream, a massage cream, essence, an eye cream, a cleansing cream, a cleansing foam, a cleansing water, a pack, a spray, or a powder. In this connection, the accessibility of the cosmetic composition may be further improved, and the chronic recurrence of atopic dermatitis may be prevented by implementing a moisturizing effect.

When the formulation of the cosmetic composition is a paste, a cream or a gel, animal oil, vegetable oil, wax, paraffin, starch, tragacanth, cellulose derivatives, polyethylene glycol, silicone, bentonite, silica, talc, zinc oxide or the like may be used as the carrier ingredient.

When the formulation of the cosmetic composition is a powder or a spray, lactose, talc, silica, aluminum hydroxide, calcium silicate, or polyamide powders may be used as the carrier ingredient, and in particular, when the formulation is a spray, a propellent such as chlorofluorohydrocarbon, propane/butane or dimethyl ether may be contained.

When the formulation of the cosmetic composition is a solution or an emulsion, a solvent, a solubilizing agent or an emulsifier may be used as the carrier ingredient, and examples thereof include water, ethanol, isopropanol, ethyl carbonate, ethyl acetate, benzyl alcohol, benzyl benzoate, propylene glycol, 1,3-butylglycol oil, glycerol aliphatic esters, polyethylene glycol or sorbitan fatty acid esters.

When the formulation of the cosmetic composition is a suspension, a liquid diluent such as water, ethanol and propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar, tragacanth or the like may be used as the carrier ingredient.

When the formulation of the cosmetic composition is a surfactant-containing cleanser, aliphatic alcohol sulfate, aliphatic alcohol ether sulfate, sulphosuccinic acid monoester, isethionate, imidazolinium derivative, methyltaurate, sarcosinate, fatty acid amide ether sulfate, alkylamidobetain, aliphatic alcohol, fatty acid glyceride, fatty acid diethanolamide, vegetable oils, a lanolin derivative or ethoxylated glycerol fatty acid ester or the like may be used as the carrier ingredient.

When the cosmetic composition is in the form of a soap, it may be prepared by comprising a skin moisturizer, an emulsifier, a water softener, etc. as an additive. As a base material of the soap, vegetable oils such as coconut oil, palm oil, soybean oil, olive oil, palm kernel oil, jojoba, etc., or animal fats such as beef tallow, pork lard, brisket, fish oil, etc. may be used. As a moisturizer, glycerin, detipritol, propylene glycol, butylene glycol, hexyl glycol, isopropyl myristate, aloe vera, sorbitol, etc. may be used. As an emulsifier, natural oil, wax, hydrocarbons, etc. may be used, and as a water softener, tetrasodium EDTA, etc. may be used, but is not limited thereto.

The soap may further include an antibacterial agent, a foam inhibitor, a solvent, a corrosion inhibitor, a fragrance, a colorant, a sequestering agent, a preservative, and the like as additives.

When the composition of the present invention is used as a cosmetic composition, the 17-HDHA, Resolvin D5, and Protectin DX in the composition may be contained in a concentration of 30 µM or more, specifically 35 µM or more, more specifically 40 µM or more. However, this varies depending on the form in which the cosmetic composition is prepared and its specific application site (face or hand) or application dose, and thus is not limited thereto.

Yet another aspect of the present invention provides a pharmaceutical composition for preventing or treating a skin disease, wherein the pharmaceutical composition contains 17-hydroxydocosahexaenoic acid (17-HDHA), Resolvin D5, and Protectin DX as active ingredients.

The skin disease may be a skin wound, skin scar, dermatitis, atopic dermatitis, pruritus, eczematous skin disease, dry eczema, athlete's foot, erythema, urticaria, psoriasis, weak rash, acne or hair loss.

The complex composition containing the 17-hydroxydocosahexaenoic acid (17-HDHA), Resolvin D5, and Protectin DX, and the content related to skin diseases are the same as described above.

When the composition is used as a pharmaceutical composition, the composition may further include a pharmaceutically acceptable carrier or additive, in addition to the 17-HDHA, Resolvin D5, and Protectin DX.

The "pharmaceutically acceptable" means that an object to be applied (prescribed) does not have toxicity beyond adaptable without inhibiting the activity of an active ingredient. The "carrier" is defined as a compound that facilitates the addition of the compound into cells or tissues.

The 17-HDHA, Resolvin D5, and Protectin DX of the present invention may be administered alone or in combination with any convenient carrier, etc., and such a dose formulation may be a single-dose or repeat-dose formulation. The pharmaceutical composition may be a solid formulation or a liquid formulation. The solid formulation includes powders, granules, tablets, capsules, and suppositories, but is not limited thereto. The solid formulation may include a carrier, a flavoring agent, a binder, a preservative, a disintegrant, a lubricant, a filler, and the like, but is not limited thereto. The liquid formulation includes solutions such as water and propylene glycol solutions, suspensions, and emulsions, but is not limited thereto, and may be prepared by adding suitable coloring agents, flavoring agents, stabilizers, viscosifying agents, etc. For example, the powders may be prepared by simply mixing a suitable pharmaceutically acceptable carrier such as lactose, starch, microcrystalline cellulose, etc. with the 17-HDHA, Resolvin D5, and Protectin DX, which are the active ingredients of the present invention. The granules may be prepared by mixing the 17-HDHA, Resolvin D5, and Protectin DX of the present invention, a suitable pharmaceutically acceptable carrier, and a suitable pharmaceutically acceptable binder such as polyvinylpyrrolidone, and hydroxypropyl cellulose, and then using a wet granulation method using a solvent such as water, ethanol, and isopropanol or a dry granulation method using compressive force. In addition, the tablets may be prepared by mixing the granules with a suitable pharmaceutically acceptable lubricant such as magnesium stearate, and then tableting the mixture using a tablet machine.

The 17-HDHA, Resolvin D5, and Protectin DX of the present invention may be administered with an oral agent, injections (e.g., intramuscular injection, intraperitoneal injection, intravenous injection, infusion, subcutaneous injection, and implant), an inhalation agent, a nasal administering agent, a vaginal agent, a rectal administering agent, a sublingual agent, a transdermal agent, a topical agent, or the like depending on a disease to be treated and a condition of a subject, but is not limited thereto. The derivatives may be formulated in a suitable dosage unit formulation comprising a pharmaceutically acceptable carrier, an additive, and a vehicle, which are conventionally used according to a route of administration and non-toxic.

The pharmaceutical composition of the present invention may be administered at a daily dose of about 0.0001 mg/kg to about 10 g/kg, and about 0.001 mg/kg to about 1 g/kg. However, the dose may vary depending on a degree of purification of the mixture, a patient's condition (age, gender, weight, etc.), and the severity of the condition being treated. If necessary, for convenience, the total daily dose may be divided and administered several times during a day.

When the composition of the present invention is used as a pharmaceutical composition, the 17-HDHA, Resolvin D5, and Protectin DX in the composition may be contained in a concentration of 30 µM or more, specifically 35 µM or more, more specifically 40 µM or more.

Yet another aspect of the present invention provides a food composition for improving skin condition, wherein the food composition contains 17-hydroxydocosahexaenoic acid (17-HDHA), Resolvin D5, and Protectin DX as active ingredients.

The complex composition containing the 17-hydroxydocosahexaenoic acid (17-HDHA), Resolvin D5, and Protectin DX, and the content related to skin condition improvement are the same as described above.

When the composition is used as a food composition, the composition may contain acceptable food supplement additives, and may further include suitable carriers, excipients, and diluents commonly used in the preparation of food.

In the present invention, the food refers to a natural product or processed product containing one or more nutrients, and specifically, refers to a state that may be eaten directly through a certain processing process, and as a general meaning, is used as a meaning comprising all of various foods, functional foods, beverages, food additives, and beverage additives. Examples of the food include various foods, beverages, gum, tea, vitamin complexes, functional foods, and the like. In addition, the food of the present invention includes special nutritional foods (e.g., formulas, infant foods, etc.), processed meat products, fish meat products, tofu, muk, noodles (e.g., ramen, noodles, etc.), health supplement foods, seasoning foods (e.g., soy sauce, soybean paste, red pepper paste, mixed sauce, etc.), sauces, confectionery (e.g., snacks), dairy products (e.g., fermented milk, cheese, etc.), other processed foods, kimchi, salted foods (various kimchi, pickles, etc.), beverages (e.g., fruit, vegetable beverages, soy milk, fermented beverages, ice cream, etc.), natural seasonings (e.g., ramen soup, etc.), vitamin complexes, alcoholic beverages, liquors, and other health supplements, but is not limited thereto. The functional foods, beverages, food additives or beverage additives may be prepared by general preparation methods.

The term "functional food" refers to food that is designed and processed to sufficiently express a body regulation function on the body with respect to biological defense rhythm control, disease prevention and recovery, etc. of a food group or food composition that has an added value to act and express the function of the corresponding food for a specific purpose by using physical, biochemical, and biotechnological methods, etc., and specifically, may be a health functional food.

The term "health functional food" used in the present invention refers to food prepared and processed in the form of tablets, capsules, powders, granules, liquids and pills by using raw materials or ingredients having functionalities useful to the human body. Here, the "function" refers to adjusting nutrients to the structure and function of the human body or to obtaining effects useful for health applications such as physiological action. The health functional food of the present invention may be prepared by methods which are commonly used in the art and may be prepared by adding raw materials and ingredients which are commonly added in the art in preparation. In addition, the formulation of the health functional food may also be prepared without limitation as long as the formulation is recognized as a health functional food. The food composition of the present invention may be prepared in various types of formulations, and unlike general drugs, the food composition has an advantage that there is no side effect that may occur when taking the drug in a long term by using the food as a raw material, and has excellent portability, but the health functional food of the present invention may be taken as supplements for enhancing a skin improvement effect.

In addition, the functional food may include sitologically acceptable food supplement additives, and may further include suitable carriers, excipients, and diluents commonly used in the preparation of functional foods.

In addition, in the food composition, the 17-HDHA, Resolvin D5, and Protectin DX in the composition may be contained in a concentration of 30 µM or more, specifically 35 µM or more, more specifically 40 µM or more.

The food composition of the present invention may contain sweetening agents, flavoring agents, physiologically active ingredients, minerals, etc., in addition to the active ingredients. The sweetening agent may be used in an amount to give a suitable sweet taste of the food, and may be natural or synthetic. Specifically, when a natural sweetening agent is used, examples of the natural sweetening agent may include sugar sweetening agents such as corn syrup solids, honey, sucrose, fructose, lactose, and maltose. The flavoring agent may be used to improve taste or flavor, and both natural and synthetic flavoring agents may be used. Specifically, the natural flavoring agents are used. In the case of using the natural flavoring agents, the purpose of nutrient enhancement may be combined in addition to flavoring. The natural flavoring agents may be obtained from apples, lemons, tangerines, grapes, strawberries, peaches, and the like, or obtained from green tea leaves, solomon's seal, bamboo leaves, cinnamon, chrysanthemum leaves, jasmine, and the like. In addition, flavoring agents obtained from ginseng (red ginseng), bamboo shoots, aloe vera, and ginkgo may be used. The natural flavoring agents may be liquid concentrates or solid extracts. In some cases, the synthetic flavoring agents may be used, and the synthetic flavoring agents may use esters, alcohols, aldehydes, terpenes, and the like. As the physiologically active ingredients, catechins such as catechin, epicatechin, gallocatechin, and epigallocatechin, and vitamins such as retinol, ascorbic acid, tocopherol, calciferol, thiamine, and riboflavin may be used. As the minerals, calcium, magnesium, chromium, cobalt, copper, fluoride, germanium, iodine, iron, lithium, magnesium, manganese, molybdenum, phosphorus, potassium, selenium, silicon, sodium, sulfur, vanadium, zinc, and the like may be used.

In addition, the food composition of the present invention may contain a preservative, an emulsifier, an acidulant, a thickener, and the like, if necessary, in addition to the sweetening agent, and the like. These preservatives, emulsifiers, and the like are preferably added and used in a very trace amount as long as the application to be added may be achieved. The trace amount means a range of about 0.0005 wt% to about 0.5 wt% based on the total weight of the food composition when expressed numerically. Preservatives that may be used may include sodium calcium sorbate, sodium sorbate, potassium sorbate, calcium benzoate, sodium benzoate, potassium benzoate, ethylenediaminetetraacetic acid (EDTA), and the like. Examples of the emulsifier that may be used may include acacia gum, carboxymethylcellulose, xanthan gum, pectin, and the like. Examples of the acidulant that may be used may include citric acid, malic acid, fumaric acid, adipic acid, phosphoric acid, gluconic acid, tartaric acid, ascorbic acid, acetic acid, phosphoric acid, etc. These acidulants may be added so that the food composition has an appropriate acidity for the purpose of inhibiting the proliferation of microorganisms in addition to the purpose of enhancing taste. As the thickening agent that may be used, a suspending agent, a settling agent, a gel-forming agent, a swelling agent, and the like may be included.

Hereinafter, the present invention will be described in detail by Examples.

However, the following Examples are specifically illustrative of the present invention, and the contents of the present invention are not limited to the following Examples.

### [Example 1]

### Preparation of Protein Comprising Amino Acid Sequence represented by SEQ ID NO: 1 or 2

### [1-1] Preparation of Vector for Expressing Protein

A nucleotide sequence represented by SEQ ID NOs: 3 and 4 encoding each of amino acids of SEQ ID NOs: 1 and 2 were synthesized by requesting Bioneer Co., Ltd. Pre-denaturation was performed at 94°C for 5 minutes using primer pairs of SEQ ID NOs: 5 to 8 using the respective synthesized nucleotide sequence as a template, and then PCR was performed by repeating cycles 20 times of reacting at 94°C for 30 seconds, at 61°C for 30 seconds, and at 72°C for 2 minutes to amplify each gene of SEQ ID NOs: 3 and 4.

**[Table 1]**

| Proteins | Primer nucleotide sequences(5'->3') | | SEQ ID NOs |
|---|---|---|---|
| Amino acid of SEQ ID NO: 1 | Forward | CATATGATGTTTGGCATCTTCGACAAG G | 5 |
| | Revere | CTCGAGTTAGATAGAGATACTGTTCGG GATCCCC | 6 |
| Amino acid of SEQ ID NO: 2 | Forward | CATATGATGACAGGTGGATGTTTGGA C | 7 |
| | Revere | CTCGAGTTAGATGGAAATACTGTTGGG AATTCCTTTG | 8 |

PCR products containing the nucleotide sequence represented by SEQ ID NOs: 3 and 4 amplified as described above were inserted into a plasmid vector pET28a(+) (Novagen, USA), respectively, to prepare a recombinant expression vector. It was identified that the nucleotide sequence represented by SEQ ID NOs: 3 and 4 were properly inserted through nucleotide sequencing analysis (Solgent).

### [1-2] Expression and Purification of Protein

The recombinant expression vector prepared in Example [1-1] was transformed into *E. coli* BL21 (DE3), and the respective transformant was inoculated into 3 ml of an LB medium, and seed-cultured at 37°C until the absorbance at 600 nm became 2.0. Then, a main culturing was performed by adding the seed-incubated culture medium to 500 ml of the LB medium. When the absorbance at 600 nm became 0.6, IPTG (isopropyl-1-thio-β-D-galactopyranoside) was added to a final concentration of 1 mM to induce the overexpression of the protein having the amino acid sequence represented by SEQ ID NO: 1 and the protein having the amino acid sequence represented by SEQ ID NO: 2. In the process of inducing overexpression as described above, the culture temperature was maintained at 37°C before adding IPTG, and the culture temperature was lowered to 20°C after adding IPTG.

A supernatant was isolated by centrifuging the culture medium of the transformant in which the overexpression was induced, and cells of the transformant were lyzed from a pellet in which the supernatant was isolated to obtain a cell lysate of the transformant.

As a result of performing SDS-PAGE on 100 µl of the cell lysate obtained as described above, as illustrated in FIG. 1, it was identified that a protein having an amino acid sequence represented by SEQ ID NO: 1 with a size of about 96.9 KDa (FIG. 1A) and a protein having an amino acid sequence represented by SEQ ID NO: 2 with a size of about 96.8 KDa (FIG. 1B) were overexpressed.

With respect to the cell lysate identified that the protein having the amino acid sequence represented by SEQ ID NO: 1 and the protein having the amino acid sequence represented by SEQ ID NO: 2 as described above were overexpressed, the protein having the amino acid sequence represented by SEQ ID NO: 1 and the protein having the amino acid sequence represented by SEQ ID NO: 2 were isolated and purified using Ni-NTA adsorption chromatography.

### [Example 2]

### Identification of Protein Activity - Identification of Production Activity of Mono- or Di-Hydroxy Derivatives

With respect to the protein having the amino acid sequence represented by SEQ ID NO: 1 and the protein having the amino acid sequence represented by SEQ ID NO: 2, purified and isolated in Example [1-2], each of 6 KU or 10 KU of the protein and 100 µM of docosahexaenoic acid (DHA) was reacted at pH 7 and room temperature for 30 minutes, a reaction product was reduced by adding 1 M of sodium borohydride so that the final concentration became 50 mM, and then the reaction was terminated by adding 5 µl/ml of acetic acid.

After the reaction product was purified using a solid phase cartridge (SPE, C18 500 mg), the types of compounds in the reaction product were analyzed using normal phase HPLC.

Specifically, the normal phase HPLC analysis was performed by developing 20 ml of a mobile phase consisting of 95% n-heptane, 5% isopropanol, 0.1% acetic acid, and 0.1% 2,2-dimethoxypropane on a supelcosil LC-Diol column (Supelco, 25 cm × 3 mm, 5 µm) at a flow rate of 0.5 ml/min for 40 minutes, and finally detecting the compounds in the reaction product using a diode array detector (DAD).

As a result, as illustrated in FIGS. 2 and 3, it was identified that both the protein having the amino acid sequence represented by SEQ ID NO: 1 and the protein having the amino acid sequence represented by SEQ ID NO: 2 could produce 17S-monohydroxy-DHA, 7S,17S-di-hydroxy-DHA (Resolvin D5), and 10S,17S-dihydroxy-DHA (Protectin DX) from DHA. In each case, it was identified that the contents of the above three mono- or di-hydroxy derivatives existed at ratios as described in Table 2 below, respectively.

**[Table 2]**

| **Enzymes** | **Secondary structures** | **Contents (ratios)** |
|---|---|---|
| Derivatives produced by a protein having the amino acid sequence represented by SEQ ID NO: 1 | 17S-monohydroxy -DHA | 1.03 mg (25.06%) |
| | 7S, 17S-di-hydroxy-DHA (Resolvin D5) | 2.43 mg (59.04%) |
| | 10S, 17S-dihydroxy-DHA (Protectin DX) | 0.66 mg (15.98%) |
| Derivatives produced by a protein having the amino acid sequence represented by SEQ ID NO: 2 | 17S-monohydroxy-DHA | 0.53 mg (20.98%) |
| | 7S, 17S-di-hydroxy-DHA (Resolvin D5) | 1.79 mg (71.45%) |
| | 10S, 17S-dihydroxy-DHA (Protectin DX) | 0.19 mg (7.44%) |

### [Example 3]

### Amino Acid Sequence Analysis of Protein

With respect to the protein having the amino acid sequence represented by SEQ ID NO: 1 and the protein having the amino acid sequence represented by SEQ ID NO: 2, as compared to human 5LOX (AAA36183), human 12LOX (AAA51533), human 15LOX (AAA36183), soybean 15LOX (AAA33986), potato LOX (AAB81595), and red algae PhLOX2 (AGN54275), homology analysis and system analysis of amino acid sequences were performed.

As a result, as illustrated in FIGS. 4A to 4D, it was identified that the protein having the amino acid sequence represented by SEQ ID NO: 1 and the protein having the amino acid sequence represented by SEQ ID NO: 2 were found to exhibit very low sequence homology with human 5LOX (AAA36183), human 12LOX (AAA51533), human 15LOX (AAA36183), soybean 15LOX (AAA33986), potato LOX (AAB81595), and red algae PhLOX2 (AGN54275).

In addition, as may be understood from FIGS. 4A to 4D, it was identified that both the protein having the amino acid sequence represented by SEQ ID NO: 1 and the protein having the amino acid sequence represented by SEQ ID NO: 2 had an N-terminal region for binding to the membrane lipid, and showed high similarity in the C-terminal region. In particular, it was identified that residues binding to iron ions, such as His, His, His and Asn/Ser, and terminal amino acids were present, and Ala, which was closely associated with the hydroxylation properties of the enzyme, was present.

In addition, as a result of analyzing a secondary structure of the protein having the amino acid sequence represented by SEQ ID NO: 1 and the protein having the amino acid sequence represented by SEQ ID NO: 2, it was identified that α-helixes, extended strands, and random coils existed at ratios shown in Table 3 below, respectively.

**[Table 3]**

| **Enzymes** | **Secondary structures** | **Ratios** |
|---|---|---|
| Protein having the amino acid sequence represented by SEQ ID NO: 1 | α-helixes | 33.14% |
| | extended strands | 26.33% |
| | random coils | 40.53% |
| Protein having the amino acid sequence represented by SEQ ID NO: 2 | α-helixes | 31.83% |
| | extended strands | 29.17% |
| | random coils | 39.00% |

### [Example 4]

### Identification of Effect of Reaction Product Produced by Enzyme Activity of Protein of Present Invention - Inhibition of Skin Aging and Wrinkle Alleviation

As in Example 2 above, the product obtained by reacting docosahexaenoic acid with the protein having the amino acid sequence represented by SEQ ID NO: 1 of the present invention (SPM 1) and the product obtained by reacting docosahexaenoic acid with the protein having the amino acid sequence represented by SEQ ID NO: 2 (SPM 2) was treated with human dermal fibroblasts to identify cytotoxicity, MMP-1 expression and procollagen synthesis effects, and was treated with human keratinocytes to identify changes in cytotoxicity, TNF-α expression and IL-6 expression.

### [4-1] Identification of cytotoxicity

Cell viability was identified by treating human dermal fibroblasts and human keratinocytes with the SPM 1 and SPM 2 at concentrations of 0.001, 0.01, 0,1, and 1 ppm, respectively.

As a result, as illustrated in FIGS. 5 and 6, both reaction products (SPM 1, SPM 2) exhibited cell viability of 90% or more at all concentrations, and therefrom, it may be understood that the product produced from DHA by the protein of the present invention are all non-cytotoxic.

### [4-2] Identification of TNF-α and IL-6 expression inhibitory activity improvement effect

Human keratinocytes were aliquoted in a 48-well plate at a concentration of 4 × 10⁴ cells/well and cultured for 24 hours at 5% CO₂ and 37°C, then the medium was removed and starvation state was maintained with DMEM Serum Free media for 24 hours.

The SPM 1 and SPM 2 were diluted with DMEM (2% FBS) to make 1 ppm, and then diluted sequentially to 0.1, 0.01 and 0.001 ppm, and then cultured as above and treated with human keratinocytes from which the medium was removed for 1 hour. Then, for TNF-α or IL-6 expression, the medium was exchanged with 250 µl of DPBS (WelGENE), irradiated with 160 mJ/cm² of UVB, and then treated with the SPM 1 and SPM 2 again and cultured for 24 hours. In addition, after collecting the medium, the amount of TNF-α was measured using Human TNF-α DuoSet ELISA (R&D system), and the amount of IL-6 was measured using Human IL-6 DuoSet ELISA (R&D system), respectively. The cells adhering to the bottom were washed with DPBS, dissolved with 1N NaOH, and the amount of protein was measured through BCA analysis to measure the amount of TNF-α or IL-6 synthesized per a given protein. In this connection, the effect of the two reaction products SPM 1 and SPM 2 was identified by using a case in which nothing was treated as a negative control group and a case in which 10 ppm of dexamethasone was treated as a positive control group.

First, in the case of TNF-α, as illustrated in FIG. 7, the expression of TNF-α was increased by about 2 times by irradiating UVB. This increased expression of TNF-α was reduced by about 35% by treatment with 10 ppm of dexamethasone, while it is reduced by about 24% by treatment with 1 ppm of SPM 1, and by about 56% by treatment with 1 ppm of SPM 2. The expression inhibitory effect of TNF-α showed a tendency dependent on the treatment concentration of SPM 1 and SPM 2.

Next, in the case of IL-6, as illustrated in FIG. 8, the expression of IL-6 was increased by about 1.6 times by irradiating UVB. This increased expression of IL-6 was reduced by about 88% by treatment with 10 ppm of dexamethasone, while it is reduced by about 35% by treatment with 1 ppm of SPM 1, and by about 92% by treatment with 1 ppm of SPM 2. The expression inhibitory effect of IL-6 also showed a tendency dependent on the treatment concentration of SPM 1 and SPM 2.

From the above results, it may be understood that all products produced from DHA by the protein of the present invention have an effect of terminating inflammation.

### [4-3] Identification of procollagen synthesis ability improvement effect

Collagen, a major component of the extracellular matrix, is synthesized in the form of a precursor called procollagen. The procollagen is known to be cut and isolated from collagen molecules when a polymerization reaction occurs. By measuring the amount of procollagen, the degree of collagen biosynthesis in the cells may be estimated.

Human dermal fibroblasts were treated with SPM 1 and SPM 2 at concentrations of 0.01, 0,1 and 1 ppm, respectively, and cultured for 24 hours, and then the cultured cell medium was collected and procollagen typeI c-peptide (PIP) EIA kit (TAKARA, MK101) was used to measure the amount of procollagen. In this connection, the effect of the two reaction products SPM 1 and SPM 2 was identified by using a case in which nothing was treated as a negative control group and a case in which 26.2 ppm of retinyl palmitate was treated as a positive control group.

As a result, as illustrated in FIG. 9, both reaction products (SPM 1 and SPM 2) were identified to have a greater amount of procollagen than the negative control group that was not treated with anything. In particular, in the case of SPM 2, a greater amount of procollagen was identified compared to the positive control group (retinyl palmitate at 26.2 ppm) at a concentration of 0.1 ppm or more. Therefrom, it may be understood that all products produced from DHA by the protein of the present invention improve the synthesis ability of procollagen.

### [4-4] Identification of Collagenase (MMP-1) Expression Inhibitory Ability Improvement Effect

MMPs (matrix metalloproteinases) are enzymes with proteolytic activity, and by decomposing proteins constituting the extracellular matrix, they have an important effect on the binding of cells and proteins constituting the extracellular matrix. There are 28 types of MMPs, of which MMP-1 is an enzyme that decomposes collagen. In this example, the expression level of MMP-1 in cells was identified.

After culturing human dermal fibroblasts for 24 hours, the medium was exchanged with DPBS (WelGENE) at a concentration of 250 µl/well, and irradiated with 100 mJ/cm² of UVB. Then, the SPM 1 and SPM 2 were treated with DMEM (2% FBS) diluted to a concentration of 0.01, 0,1 and 1 ppm, respectively, and cultured for 48 hours. After collecting the cultured cell medium, the amount of MMP-1 was measured using a human total MMP-1 ELISA kit (R&D systems, DY901). At this connection, the effect of the two reaction products SPM 1 and SPM 2 was identified by using a case in which nothing was treated as a negative control group and a case in which 6 ppm of retinoic acid was treated as a positive control group.

As a result, as illustrated in FIG. 10, the expression of MMP-1 was increased by about 2 times by irradiating UVB. This increased expression of MMP-1 was reduced by about 70% by treatment with 6 ppm of retinoic acid, while it is reduced by about 17.2% and 80.1%, respectively, by treatment with 0.1 ppm and 1 ppm of SPM 1, and by 11.4% by treatment with 0.01 ppm of SPM 2, 26.1% by treatment with 0.1 ppm of SPM 2, and 63.3% by treatment with 1 ppm of SPM 2. The expression inhibitory effect of MMP-1 also showed a tendency dependent on the treatment concentration of SPM 1 and SPM 2.

### [Example 5]

### Production of SPMs Complex Compositions and Preparation of Single Compounds

### [5-1] Complex Composition of 17-HDHA, Resolvin D5 and Protectin DX (hereinafter, referred to as 'SPMs complex composition')

Lipoxygenase having the amino acid sequence represented by SEQ ID NO: 1 was added to various concentrations of docosahexaenoic acid (DHA) and reacted at pH 7 and room temperature for 30 minutes. The reaction product was reduced by adding 1 M of sodium borohydride so that the final concentration became 50 mM, and then the reaction was terminated by adding 5 µl/ml of acetic acid. After the reaction product was purified using a solid phase cartridge (SPE, C18 500 mg), types of compounds in the reaction product were analyzed using normal phase HPLC.

As a result, it was identified that 17S-HDHA, Resolvin D5 (7S, 17S-diHDHA), and Protectin DX (10S, 17S-diHDHA) were produced from DHA. Therefrom, Complexes 1 to 3, which are SPMs complex compositions present in a mixed state in a ratio as described in Table 4 below, respectively, were produced.

**[Table 4]**

| | **Complex 1** | **Complex 2** | **Complex 3** |
|---|---|---|---|
| **17S-HDHA** | 3 | 25 | 84 |
| **Resolvin D5** | 47 | 56 | 10 |
| **Protectin DX** | 50 | 19 | 6 |

### [5-2] Isolation and purification of 17-HDHA, Resolvin D5 and Protectin DX

17S-HDHA, Resolvin D5 (7S, 17S-diHDHA), and Protectin DX (10S, 17S-diHDHA) were used by isolating and purifying each product from the SPMs complex composition of Example [5-1] above through prep HPLC equipped with a C18 column or a Diol column.

### [Example 6]

### Identification of Synergistic IL-6 Expression Inhibition Effect by SPMs Complex Composition

In order to identify the synergistic antiinflammation relieving effect of the SPMs complex composition, the complex compositions (Complexes 1 to 3) of 17-HDHA, Resolvin D5, and Protectin DX and each SPM single material were treated with HaCaT cells, which are human keratinocytes to identify changes in IL-6 expression.

### [6-1] Experimental Method

Specifically, first, human keratinocytes were aliquoted in a 48-well plate at a concentration of 4 × 10⁴ cells/well and cultured at 5% CO2, 37°C for 24 hours, and then the medium was removed and the starvation state was maintained with DMEM Serum Free media for 24 hours.

The SPMs complex composition was diluted with DMEM (FBS 2%) to make 0.5, 1, 2, and 5 µg/ml, and then cultured as above and treated with human keratinocytes from which the medium was removed for 1 hour. Then, for IL-6 expression, the medium was exchanged with 250 µl of DPBS (WelGENE), irradiated with 160 mJ/cm² of UVB, and then treated with SPMs again and cultured for 24 hours. In addition, after collecting the medium, the amount of IL-6 was measured using Human IL-6 DuoSet ELISA (R&D system). The cells adhering to the bottom were washed with DPBS, dissolved with 1N NaOH, and the amount of protein was measured through BCA analysis to measure the amount of IL-6 synthesized per a given protein. In this connection, the IL-6 expression inhibitory effect of the SPMs complex composition of the present invention and each single component was identified by using a case in which nothing was treated as Con(-) and a case in which 10 µM of hydrocortisone was treated as Con(+) after UVB irradiation.

### [6-2] Identification of IL-6 Inhibitory Effect of SPM Single Component

As a result, as shown in Table 5 and FIG 11A, the expression of IL-6 was increased by irradiating human keratinocytes with UVB. This increased expression of IL-6 was reduced by 10.8%, 11.8%, and 0.5%, respectively, compared to Con(-) when 2 µg/ml of 17S-HDHA, Protectin DX and Resolvin D5 were treated, respectively, and showed a tendency dependent on the treatment concentration of SPM.

Therefrom, it may be understood that 17-HDHA, Protectin DX, and Resolvin D5 all have inflammation relieving efficacy.

**[Table 5]**

| **Samples** | **UVB** | **Concentratio ns (µg/ml)** | **IL-6 expression (% of control)** |
|---|---|---|---|
| | | | **Average** |
| Untreated control | - | - | 23.64 |
| Con(-) | + | - | 100 |
| Con(+) H.C. | + | 10µM | 31.23 |
| 17S-HDHA | + | 0.1 | 89.63 |
| | + | 1 | 83 |
| | + | 2 | 89.17 |
| | + | 5 | 81.66 |
| PDX | + | 0.1 | 95.36 |
| | + | 1 | 102.69 |
| | + | 2 | 88.13 |
| | + | 5 | 86.72 |
| RvD5 | + | 0.1 | 99.58 |
| | + | 1 | 109.86 |
| | + | 2 | 99.49 |
| | + | 5 | 83.38 |

### [6-3] Identification of IL-6 Inhibitory Effect of SPMs Complex Composition

In addition, as shown in Table 3 and FIG 1B, the expression of IL-6 was increased by irradiating the SPMs complex composition with UVB. This increased expression of IL-6 was reduced by about 18%, 33.8%, and 29.5%, respectively, compared to Con(-) when 2 µg/ml of the SPMs complex compositions (compositions 1, 2, and 3) of the present invention were treated, and showed a tendency dependent on the treatment concentration of SPM.

Therefrom, it may be understood that the SPMs complex compositions of the present invention all have excellent inflammation relieving efficacy.

**[Table 6]**

| **Samples** | **UVB** | **Concentrations (µg/ml)** | **IL-6 expression (% of control)** |
|---|---|---|---|
| | | | **Average** |
| Untreated control | - | - | 15.62 |
| Con(-) | + | - | 100 |
| Con(+) H.C. | + | 10µM | 42.82 |
| Composition 1 (17S-HDHA : RvD5 : PDX = 3 : 47 : 50) | + | 0.1 | 74.66 |
| | + | 1 | 72.49 |
| | + | 2 | 72.98 |
| | + | 5 | 64.93 |
| Composition 2 (17S-HDHA : RvD5 : PDX = 25 : 56 : 19) | + | 0.1 | 71.28 |
| | + | 1 | 64.98 |
| | + | 2 | 66.18 |
| | + | 5 | 62.03 |
| Composition 3 (17S-HDHA : RvD5 : PDX = 84 : 10 : 6) | + | 0.1 | 80.61 |
| | + | 1 | 78.63 |
| | + | 2 | 70.52 |
| | + | 5 | 68.19 |

### [6-4] Identification of Synergistic IL-6 Inhibitory Effect of SPMs Complex Composition

As shown in Table 7 and FIG. 11C below, when 2 µg/ml of the complex composition was treated, it was identified that the expression of IL-6 was reduced by 18% in composition 1, 33.8% in composition 2, and 29.5% in composition 3 compared to Con(-). When the SPM single component was treated at the same concentration, it was identified that the effect of reducing the expression of IL-6 was increased compared thereto.

**[Table 7]**

| UVB 20mJ/cm² | | | | | | |
|---|---|---|---|---|---|---|
| Con (-) | 17S-HDHA | PDX | RvD5 | Composition 1 | Composition 2 | Composition 3 |
| 100 | 89.17 | 88.13 | 99.49 | 72.98 | 66.18 | 70.52 |

Therefrom, it may be understood that the SPMs complex composition of the present invention has a significantly increased inflammatory relieving effect compared to a single component. In addition, the above results mean that due to the synergistic effect of 17S-HDHA, PDX and RvD5, the amount used may be reduced when using them as a complex composition rather than when using them alone. Therefrom, it may be understood that the potential side effects that may occur when SPM is used in excess may be prevented.

### [Example 7]

### Identification of Synergistic Procollagen Synthesis Effect by SPMs Complex Composition

Collagen, a major component of the extracellular matrix, is synthesized in the form of a precursor called procollagen. The procollagen is known to be cut and isolated from collagen molecules when a polymerization reaction occurs. By measuring the amount of procollagen, the degree of collagen biosynthesis in the cells may be estimated.

### [7-1] Experimental Method

Human dermal fibroblasts were treated with the SPM single material and its complex compositions (compositions 1 to 3) at concentrations of 0.1, 1, 2, and 5 µg/ml, respectively, and cultured for 24 hours, and then the cultured cell medium was collected and procollagen typeI c-peptide (PIP) EIA kit (TAKARA, MK101) was used to measure the amount of procollagen. In this connection, the effect of the SPM single component and its complex compositions (compositions 1 to 3) was identified by using a case in which nothing was treated as an untreated control group and a case in which 50 µM of retinyl palmitate was treated as Con(+).

### [7-2] Identification of Procollagen Synthesis Effect of SPM single component

As shown in Table 8 and FIG. 12A below, all of 17-HDHA, Protectin DX, and Resolvin D5 were identified to have a greater amount of procollagen than the untreated control group. In particular, in Protectin DX and Resolvin D5, a greater amount of procollagen was identified compared to the positive control group (50 µM of retinyl palmitate) at a concentration of 1 µg/ml or more.

Therefrom, it may be understood that 17-HDHA, Protectin DX, and Resolvin D5 all improve the synthesis ability of procollagen.

**[Table 8]**

| **Samples** | **Concentrations (µg/ml)** | **Procollagen synthesis (% of control)** |
|---|---|---|
| | | **Average** |
| Untreated control | - | 100 |
| Con(+) R.P. | 50 µM | 249.88 |
| 17S-HDHA | 0.01 | 212.76 |
| | 0.1 | 237.76 |
| | 1 | 235.8 |
| | 2 | 255.32 |
| PDX | 0.01 | 237.15 |
| | 0.1 | 244.1 |
| | 1 | 251.41 |
| | 2 | 254.1 |
| RvD5 | 0.01 | 245.68 |
| | 0.1 | 248 |
| | 1 | 249.34 |
| | 2 | 255.32 |

### [7-3] Identification of Procollagen Synthesis Effect of SPMs Complex Composition

In addition, in the case of the SPMs complex compositions, as shown in Table 9 and FIG. 12B below, all of compositions 1 to 3 with different mixing ratios were identified to have 2 to 3 times or more the amount of procollagen than the untreated control group. In particular, in the SPMs complex composition, a greater amount of procollagen was identified in all of compositions 1 to 3 compared to Con(+) treated with 50 µM of retinyl palmitate at a concentration of 0.1 µg/ml or more.

Therefrom, it may be understood that all of the SPMs complex compositions of the present invention significantly improve the procollagen synthesis ability.

**[Table 9]**

| **Samples** | **Concentrations (µg/ml)** | **Procollagen synthesis (% of control)** |
|---|---|---|
| | | **Average** |
| Untreated control | - | 100 |
| Con(+) R.P. | 50 µM | 240.2 |
| Composition 1 (17S-HDHA : RvD5 : PDX = 3 : 47 : 50) | 0.01 | 218.2 |
| | 0.1 | 259.3 |
| | 1 | 289.5 |
| | 2 | 302.7 |
| Composition 2 (17S-HDHA : RvD5 : PDX = 25 : 56 : 19) | 0.01 | 225.6 |
| | 0.1 | 265.2 |
| | 1 | 302 |
| | 2 | 307.1 |
| Composition 3 (17S-HDHA : RvD5 : PDX = 84 : 10 : 6) | 0.01 | 220.7 |
| | 0.1 | 267.3 |
| | 1 | 301.1 |
| | 2 | 304.4 |

### [7-4] Identification of Synergistic Procollagen Synthesis Effect of SPMs Complex Composition

As shown in Table 10 and FIG. 12C below, all of the SPMs complex compositions (composition 1, 2, and 3) were identified to have a greater amount of procollagen than 17-HDHA, Protectin Dx, or Resolvin D5 single component.

**[Table 10]**

| Untreated control | 17S-HDHA | PDX | RvD5 | Composition 1 | Composition 2 | Composition 3 |
|---|---|---|---|---|---|---|
| 100 | 255.32 | 254.1 | 255.32 | 302.7 | 307.1 | 304.4 |

Therefrom, it may be understood that the SPMs complex composition of the present invention has a significantly increased procollagen synthesis ability_compared to a single component. In addition, the above results mean that due to the synergistic effect of 17S-HDHA, PDX and RvD5, the amount used may be reduced when using them as a complex composition rather than when using them alone. Therefrom, it may be understood that the potential side effects that may occur when SPM is used in excess may be prevented.

### [Example 8]

### Identification of Collagenase (MMP-1) Expression Inhibitory Ability Improvement Effect by SPMs Complex Composition

MMPs (matrix metalloproteinases) are enzymes with proteolytic activity, and by decomposing proteins constituting the extracellular matrix, they have an important effect on the binding of cells and proteins constituting the extracellular matrix. There are 28 types of MMPs, of which MMP-1 is an enzyme that decomposes collagen. In this example, the expression level of MMP-1 in cells was identified.

Specifically, after culturing human dermal fibroblasts for 24 hours, the medium was exchanged with DPBS (WelGENE) at a concentration of 250 µl/well, and irradiated with 100 mJ/cm² of UVB. Then, the composition 2 was treated with DMEM (2% FBS) diluted to a concentration of 0.1, 1, 2, and 5 ppm, respectively, and cultured for 48 hours. After collecting the cultured cell medium, the amount of MMP-1 was measured using a human total MMP-1 ELISA kit (R&D systems, DY901). At this connection, the effect of composition 2 was identified by using a case in which nothing was treated as a negative control group and a case in which 20 µM of retinoic acid was treated as a positive control group.

As a result, as illustrated in FIG. 13, the expression of MMP-1 was increased by about 2 times by irradiating UVB. This increased expression of MMP-1 was reduced by about 70% by treatment with 20 µM of retinoic acid, while it is reduced by 11.4% by treatment with 0.01 ppm, 26.1% by the treatment with 0.1 ppm, and 63.3% by treatment with 1 ppm of the SPMs complex composition. The expression inhibitory effect of MMP-1 also showed a tendency dependent on the treatment concentration of the SPMs complex composition.

Therefrom, it may be understood that the SPMs complex composition of the present invention is excellent in the effect of alleviating skin wrinkles.

### [Example 9]

### Identification of TNF-α Expression Inhibitory Ability Improvement Effect by SPMs Complex Composition

As a representative inflammatory cytokine, TNF-α is known to exacerbate inflammatory skin diseases when its expression amount is increased. TNF-α, a tumor necrosis factor, is an important factor that plays the most central role in many inflammatory responses, and regulation of TNF-α expression plays an important role in inflammatory diseases. Accordingly, in order to identify the inflammation relieving effect of the SPMs complex composition of the present invention, the expression pattern of TNF-α was identified.

Specifically, human keratinocytes were aliquoted in a 48-well plate at a concentration of 4 × 10⁴ cells/well and cultured for 24 hours at 5% CO₂ and 37°C, then the medium was removed and starvation state was maintained with DMEM Serum Free media for 24 hours.

The composition 2 was diluted with DMEM (2% FBS) to make 0.5, 1, 2, and 5 µg/ml, and then cultured as above and treated with human keratinocytes from which the medium was removed for 1 hour. Then, for TNF-α expression, the medium was exchanged with 250 µl of DPBS (WelGENE), irradiated with 160 mJ/cm² of UVB, and then treated with the SPMs again and cultured for 24 hours. In addition, after collecting the medium, the amount of TNF-α was measured using Human TNF-α DuoSet ELISA (R&D system), respectively. The cells adhering to the bottom were washed with DPBS, dissolved with 1N NaOH, and the amount of protein was measured through BCA analysis to measure the amount of TNF-α synthesized per a given protein. In this connection, the effect of the SPMs complex composition was identified by using a case in which nothing was treated as a negative control group and a case in which 10 µM of hydrocortisone was treated as a positive control group.

As a result, in the case of TNF-α, as illustrated in FIG. 14, the expression of TNF-α was increased by about 5 times by irradiating UVB. This increased expression of TNF-α was reduced by about 52% by treatment with 10 µM of hydrocortisone, while it is reduced by about 49% by treatment with 1 ppm of the SPMs complex composition. The expression inhibitory effect of TNF-α showed a tendency dependent on the treatment concentration of the SPMs complex composition.

Therefrom, it may be understood that the SPMs complex composition of the present invention is excellent in an effect of terminating inflammation.

### [Example 10]

### Identification of Improvement Effect in NO Production Reduction Ability by SPMs Complex Composition

Nitric oxide (NO), a type of reactive oxygen species that is known to play an important role in inducing inflammation, is a highly reactive biogenic molecule. NO, a key molecule involved in the inflammatory response, is an inflammatory mediator and accelerates the inflammatory response, further exacerbating the inflammatory response. Accordingly, the amount of NO production was identified with respect to the stimulus induced by fine dust.

Specifically, the amount of NO generated from the cells was measured using the Griess Reagent System to measure the nitrite concentration present in the cell culture medium, and the NO concentration in the culture medium was determined using a standard curve for each concentration of sodium nitrite.

As a result, as illustrated in FIG. 15, it was identified that the production of NO was reduced by 39.2 to 70% in cells treated with 0.1 to 5 ppm of the SPMs complex composition.

Therefrom, it may be understood that the SPMs complex composition of the present invention has an effect of alleviating inflammation.

### [Example 11]

### Identification of Improvement Effect in Reduction Ability of Lipid Peroxide Production by SPMs Complex Composition

Lipid peroxidation of cells is one of the mechanisms of cellular damage occurring in aging or disease states in animals and plants, and the degree of cellular damage may be identified by measuring total malondialdehyde (MDA), a product of lipid peroxidation. Accordingly, the MDA production by the SPMs complex composition was identified for the stimulus induced by fine dust.

Specifically, the amount of total malondialdehyde (MDA), a product of lipid peroxidation, was reacted with thiobarbituric acid (TBA), and the MDA-TBA adduct was measured by the TBARS method.

As a result, as illustrated in FIG. 16, it was identified that the MDA production was reduced by 19.1 to 62.7% in the cells treated with the SPMs complex composition by 0.1 to 5 ppm.

Therefrom, it may be understood that the SPMs complex composition of the present invention has an effect of inhibiting skin aging.

### [Example 12]

### Identification of Improvement Effect of Skin Barrier Improvement Ability by SPMs Complex Composition

Filaggrin, loricrin, and involucrin are known as core proteins constituting the skin barrier. Among them, filaggrin is a protein produced by decomposition of profilagrin, which constitutes keratohyalin granule (KG) present in granule cells of the epidermis, and plays an important role in the skin barrier by aggregating keratin filaments in keratinocytes. Accordingly, it was attempted to identify the skin barrier strengthening effect by identifying he expression of filaggrin and loricrin by the sample.

Human keratinocytes were cultured in DMEM medium under the same conditions as in Example 9. In order to identify the gene expression related to skin barrier improvement ability, the cultured cells were treated with the SPMs complex composition of the present invention by concentration and cultured, and then RNA was extracted and RT-PCR was performed. The amplified gene was analyzed using the Gel documentation system and Image J program.

As a result, as illustrated in FIGS. 17A and 17B, when 50 ppm of hyaluronic acid, a positive control group, was treated, the expression of filaggrin was increased by 52.6% and loricrin by 97.7%. It was identified that when 0.1 to 5 ppm of the SPMs complex composition of the present invention was treated, filaggrin was increased by 31.3 to 52.8% and loricrin increased by 31.8 to 88.5%.

Therefrom, it may be understood that the SPMs complex composition of the present invention has the effect of strengthening the skin barrier.

### [Example 13]

### Human Efficacy Evaluation using SPMs Cream

A cream (Cream S) was produced with the composition shown in Table 11 below using the SPMs complex composition. For the comparative experiment, ceramide cream (Cream C) and base cream (Cream B) were also produced with the following composition.

**[Table 11]**

| | **Cream S** | **Cream C** | **Cream B** |
|---|---|---|---|
| **SPMs complex composition** | 2.00 | - | - |
| **Ceramide AC45** | - | 1.00 | - |
| Glycerin | 5.00 | 5.00 | 5.00 |
| 1,2-hexanediol | 2.00 | 2.00 | 2.00 |
| Oil | Appropriate amount | Appropriat e amount | Appropriate amount |
| Preservative | Appropriate amount | Appropriat e amount | Appropriate amount |
| Purified water | Appropriate amount | Appropriate amount | Appropriate amount |

| | | | |
|---|---|---|---|
| (Unit: parts by weight) | | | |

### [13-1] Identification of Skin Barrier Improvement_ Efficacy by SPMs Cream

The human efficacy evaluation for skin barrier improvement was conducted on 12 women (mean age 45 years ± 5.54). The forearm was used as the test site, and the physical damage was divided into 3 sites of the test site and the control site (no application) and applied to 4 sites. Then, Cream S (SPM cream), Cream C (Ceramide cream), and Cream B (Base cream) were to be used twice a day for 1 week on each designed site, and the device measurement was performed compared to the control site (no application). Measurements and efficacy questionnaire evaluation were conducted before skin damage, immediately after skin damage, 1 day, 4 days, and 7 days after using the test product. The skin barrier improvement effect was identified by measuring TEWL (Transepidermal Water Loss) using Tewameter TM300.

As a result, as illustrated in FIG. 18A, the ceramide cream, which is most widely used as a skin barrier improvement material, has no significant improvement compared to the Base cream, whereas the SPM cream has a statistically significant skin barrier improvement effect compared to the non-applied group and the Base cream (two-tailed test utilizing two groups assuming equal variance, t-test; p < 0.034 after 4 days of use, p < 0.027 after 7 days of use). In addition, as a result of comparing the skin barrier recovery rate (%), it was identified that the SPM cream had a comparative advantage compared to the Base cream and the ceramide cream, as illustrated in FIG. 18B (Wilcoxon signed rank test; * p ≤ 0.05, ** p ≤ 0.01, *** p ≤ 0.001).

### [13-2] Identification of Atopic Dermatitis_ Improvement Efficacy by SPMs Cream

A human efficacy evaluation was conducted for relieving itching caused by dryness in 22 test subjects. After measuring the skin moisture, 11 people were randomly assigned to each of the test products Cream S and Cream C by matching the mean values between the groups to make them use each product. The forearm and dryness lesion sites were used as test sites, and device measurement and efficacy questionnaire evaluation were conducted before product use, 2 weeks after product use, and 4 weeks after product use.

The improvement effect of atopic dermatitis was identified by measuring skin moisture using Comeometer CM825, measuring skin moisture loss using Tewameter TM300, and evaluating improvement in itchiness (VAS).

### Identification of Skin Moisturizing Effect

As illustrated in FIG. 19A, it was identified that the most widely used ceramide cream for relieving dry symptoms of atopic dermatitis showed an increase in skin moisture content compared to before product use, but there was no statistical significance, whereas the SPM cream showed a statistically significant improvement efficacy in skin moisture in the lesion site (2 weeks, 4 weeks of use; < 0.014, p < 0.0115).

### Identification of Skin Barrier Improvement Effect at Lesion Site

As illustrated in FIG. 19B, also in terms of the rate of change in the amount of transdermal moisture loss, the ceramide cream showed a decrease in transdermal moisture loss compared to before product use, but there was no statistical significance, whereas the SPM cream showed statistically significant improvement efficacy in transdermal moisture loss in the lesion site (4 weeks of use; P < 0.01451).

### Identification of Itching Alleviation Effect

As illustrated in FIG. 19C, in terms of the itching alleviation effect, it was identified that the itching index was statistically significantly improved after 2 weeks and 4 weeks of use of both the SPM cream and the ceramide cream.

Therefrom, it may be understood that the SPM cream of the present invention exhibits the effects of moisturizing the skin, improving the skin barrier, and alleviating itching at an equivalent level or higher than ceramide, which is commonly used as a functional material for alleviating skin pruritus and itching and preventing moisture loss caused by xeroderma such as atopic dermatitis, and for improving skin barrier.

### [13-3] Identification of Effect of Alleviating Eye Wrinkles and Improving Skin Moisture by SPMs Cream

The human efficacy evaluation was conducted for 20 selected test subjects for alleviation of eye wrinkles and improvement of skin moisture of Cream A and Cream S. The eye area was used as the test site, and 10 test subjects were instructed to use Cream A and the remaining 10 subjects were instructed to use Cream S twice a day for 8 weeks, respectively. Before using the test product, after 4 weeks of use, and after 8 weeks of use, the effect of alleviating wrinkles and improving skin moisture was identified through device measurement on the test subjects.

Specifically, Antera 3D CS was used to measure eye wrinkles, and a Comeometer was used to measure skin moisture.

### Identification of Effect of Alleviating Eye Wrinkles

As illustrated in FIG. 20A, it was identified that the skin wrinkle alleviation rate (%) by subject of Cream A containing retinol, a functional noticed ingredient for wrinkle alleviation, was not statistically significant compared to before product use, whereas SPM cream showed significant skin wrinkle alleviation rate (%) compared to before use at 4 and 8 weeks of use (t-test (two groups assuming equal variance, two-tailed test); *, p ≤ 0.05); **, p ≤ 0.01; ***, p ≤ 0.001).

### Identification of Eye Moisturizing Effect

As illustrated in FIGS. 20B and 20C, at 8 weeks of using the SPM cream and the retinol cream, the moisture in the skin around the eyes showed a statistically significant increase, and there was no statistically significant difference between the SPM cream and the retinol cream.

Therefrom, it may be understood that the SPM cream of the present invention exhibits an effect of increasing the amount of moisture in the skin around the eyes and alleviating the skin wrinkles around the eyes at an equivalent level or higher than retinol, which is a noticed raw material for wrinkle alleviation.

As described above, while preferred examples of the present invention have been illustratively described, the scope of the present invention is not limited only to the specific examples described above, and the present invention will be able to be appropriately modified by those skilled in the art within the appended claims of the present invention.

## Claims

1. An enzyme for producing mono- or di-hydroxy derivatives of polyunsaturated fatty acids having an amino acid sequence having at least 90% homology with an amino acid sequence represented by SEQ ID NO: 1 or 2.

2. The enzyme of claim 1, wherein the polyunsaturated fatty acid is docosahexaenoic acid (DHA) or eicosapentaenoic acid (EPA).

3. A nucleic acid molecule encoding the enzyme for producing the mono- or di-hydroxy derivatives of claim 1.

4. The nucleic acid molecule of claim 3, wherein the nucleic acid molecule consists of a nucleotide sequence represented by SEQ ID NO: 3 or 4.

5. A recombinant expression vector comprising the nucleic acid molecule of claim 3.

6. A transformant in which the recombinant expression vector of claim 5 is introduced into a host cell.

7. A method for producing mono- or di-hydroxy derivatives of polyunsaturated fatty acids *in vitro,* the method comprising: reacting the enzyme of claim 1 with polyunsaturated fatty acids.

8. The method of claim 7, further comprising: recovering mono- or di-hydroxy derivatives from a reaction product of the enzyme and the polyunsaturated fatty acids.

9. The method of claim 7, wherein the reacting is performed under a condition of 10°C to 40°C and pH 4 to pH 10.

10. The method of claim 7, wherein the polyunsaturated fatty acid is docosahexaenoic acid (DHA) or eicosapentaenoic acid (EPA).

11. A method for producing mono- or di-hydroxy derivatives of polyunsaturated fatty acids *in vivo,* wherein the method comprising:
culturing the transformant of claim 6 in the presence of polyunsaturated fatty acids; and
isolating the mono- or di-hydroxy derivatives of polyunsaturated fatty acids from the cultured culture.

12. The method of claim 11, wherein the polyunsaturated fatty acid is docosahexaenoic acid (DHA) or eicosapentaenoic acid (EPA).

13. A complex composition for improving skin condition, wherein the complex composition contains 17-hydroxydocosahexaenoic acid, Resolvin D5, and Protectin DX as active ingredients.

14. The complex composition of claim 13, wherein the improvement of the skin condition is at least one kind selected from the group consisting of prevention or alleviation of skin wrinkles, prevention or alleviation of skin aging, prevention or alleviation of skin inflammation, skin regeneration, and skin barrier strengthening.

15. The complex composition of claim 13, wherein the skin condition is at least one selected from the group consisting of photoaging due to ultraviolet rays, spots, freckles, skin wounds, dermatitis, atopic dermatitis, pruritus, eczematous skin disease, dry eczema, erythema, urticaria, psoriasis, weak rash, and acne.

16. The complex composition of claim 13, wherein the composition i) promotes collagen synthesis in cells, ii) inhibits collagen decomposition, and iii) inhibits expression or activity of matrix metalloproteinase-1 (MMP-1) .

17. The complex composition of claim 13, wherein the composition has antioxidant activity.

18. The complex composition of claim 13, wherein the composition inhibits an inflammatory factor.

19. The complex composition of claim 13, wherein the composition increases expression of filaggrin and Loricrin.

20. The complex composition of claim 13, wherein the 17-hydroxydocosahexaenoic acid, Resolvin D5, and Protectin DX are contained in a weight ratio of 3 to 85 : 10 to 60 : 6 to 50.

21. The complex composition of claim 13, wherein the composition is a cosmetic composition.

22. The complex composition of claim 13, wherein the composition is a food composition.

23. A pharmaceutical composition for preventing or treating a skin disease, wherein the pharmaceutical composition contains 17-hydroxydocosahexaenoic acid (17-HDHA), Resolvin D5, and Protectin DX as active ingredients.

24. The pharmaceutical composition of claim 23, wherein the skin disease is at least one kind selected from the group consisting of skin wounds, skin scars, dermatitis, atopic dermatitis, pruritus, eczematous skin disease, dry eczema, athlete's foot, erythema, urticaria, psoriasis, weak rash, acne and hair loss.

25. A method for improving skin condition, wherein the method comprises administering an effective amount of a complex composition containing 17-hydroxydocosahexaenoic acid (17-HDHA), Resolvin D5, and Protectin DX as active ingredients to a subject in need of improvement in the skin condition.

26. A method for preventing or treating a skin disease, wherein the method includes administering an effective amount of a complex composition containing 17-hydroxydocosahexaenoic acid (17-HDHA), Resolvin D5, and Protectin DX as active ingredients to a subject in need of treatment of the skin disease.

27. A complex composition containing 17-hydroxydocosahexaenoic acid (17-HDHA), Resolvin D5, and Protectin DX as active ingredients for use in the prevention or treatment of skin diseases.
